(19) Europäisches Patentamt
European Patent Office
Office européen des brevets

(11) EP 2 833 890 B1

(12) EUROPÄISCHE PATENTSCHRIFT

(45) Veröffentlichungstag und Bekanntmachung des Hinweises auf die Patenterteilung:
**01.02.2017 Patentblatt 2017/05**

(21) Anmeldenummer: **13713912.7**

(22) Anmeldetag: **04.04.2013**

(51) Int Cl.:
***A61K 31/555*** (2006.01) ***C07F 15/02*** (2006.01)
***A61P 7/06*** (2006.01)

(86) Internationale Anmeldenummer:
**PCT/EP2013/057071**

(87) Internationale Veröffentlichungsnummer:
**WO 2013/150087 (10.10.2013 Gazette 2013/41)**

(54) **FE(III)-PYRAZIN-KOMPLEXVERBINDUNGEN ZUR BEHANDLUNG UND PROPHYLAXE VON EISENMANGELERSCHEINUNGEN UND EISENMANGELANÄMIEN**

FE(III)-PYRAZINE-COMPLEXES FOR TREATING AND PROPHYLAXIS OF ANAEMIA SYMPTOMS AND ANAEMIA

COMPLEXES DE FE(III)-PYRAZINE POUR LE TRAITEMENT ET LA PROPHYLAXIE DES CARENCES EN FER ET DES ANÉMIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.04.2012 EP 12163308**

(43) Veröffentlichungstag der Anmeldung:
**11.02.2015 Patentblatt 2015/07**

(73) Patentinhaber: **Vifor (International) AG**
**9001 St. Gallen (CH)**

(72) Erfinder:
- **BARK, Thomas**
  **CH-8050 Zürich (CH)**
- **BUHR, Wilm**
  **78465 Konstanz (DE)**
- **BURCKHARDT, Susanna**
  **CH-8049 Zürich (CH)**
- **BURGERT, Michael**
  **88045 Friedrichshafen (DE)**
- **CANCLINI, Camillo**
  **CH-9000 St. Gallen (CH)**
- **DÜRRENBERGER, Franz**
  **CH-4143 Dornach (CH)**
- **FUNK, Felix**
  **CH-8404 Winterthur (CH)**
- **GEISSER, Peter Otto**
  **CH-9014 St. Gallen (CH)**
- **KALOGERAKIS, Aris**
  **CH-8400 Winterthur (CH)**
- **MAYER, Simona**
  **CH-9055 Bühler (CH)**
- **PHILIPP, Erik**
  **CH-9320 Arbon (CH)**
- **REIM, Stefan**
  **CH-8404 Stadel Winterthur (CH)**
- **SIEBER, Diana**
  **CH-9030 Abtwil (CH)**
- **SCHMITT, Jörg**
  **CH-9425 Thal (CH)**
- **SCHWARZ, Katrin**
  **CH-9000 St. Gallen (CH)**

(74) Vertreter: **Gille Hrabal**
**Brucknerstrasse 20**
**40593 Düsseldorf (DE)**

(56) Entgegenhaltungen:
**EP-A1- 0 120 670**

- **ZHU, FENG ET AL: "Biosynthesis, characterization and biological evaluation of Fe(III) and Cu(II) complexes of neoaspergillic acid, a hydroxamate siderophore produced by co-cultures of two marine-derived mangrove epiphytic fungi", NATURAL PRODUCT COMMUNICATIONS , 6(8), 1137-1140 CODEN: NPCACO; ISSN: 1934-578X, 2011, XP009160418,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- OHKANDA ET AL: "N-Hydroxyamide-Containing Heterocycles. Part 5.1 Synthesis of Novel Hexadentate Ligands Composed of N-Hydroxy-2(1H)-pyrazinone, Aliphatic Diamine, and 1,1,1-Tris(carboxyethoxymethyl)ethane, and Properties of Their Ferric Complexes", TETRAHEDRON, ELSEVIER SCIENCE PUBLISHERS, AMSTERDAM, NL, Bd. 51, Nr. 47, 20. November 1995 (1995-11-20), Seiten 12995-13002, XP005254741, ISSN: 0040-4020, DOI: 10.1016/0040-4020(95)00839-Z
- Junko Ohkanda ET AL: "Novel Iron Sequestering Agents: Synthesis and Iron-Chelating Properties of Hexadentate Ligands Composed of 1-Hydroxy-2(1H)-pyrimidinone, ö-Amino Carboxylic Acid, and Tris(2-aminoethyl)amine", J. Org. Chem, 30. Mai 1997 (1997-05-30), Seiten 3618-3624, XP055030457, DOI: 10.1021/jo961901m Gefunden im Internet: URL:http://pubs.acs.org/doi/abs/10.1021/jo961901m [gefunden am 2012-06-20]
- J OHKANDA ET AL: "N-Hydroxyamide-Containing Heterocycles. Part 2. Synthesis and Iron(III) Complex-Forming Tendency of 1-Hydroxy-2(1H)-pyrimidinone and -pyrazinone", BULLETIN OF THE CHEMICAL SOCIETY OF JAPAN, Bd. 66, 1. Januar 1993 (1993-01-01), Seiten 841-847, XP055000884,
- Yukio Maebayashi ET AL: "Isolation and structure of red pigment from Aspergillus ochraceus Wilh", Chemical & Pharmaceutical Bulletin, 1. Januar 1978 (1978-01-01), Seiten 1320-1322, XP055030367, Gefunden im Internet: URL:http://ci.nii.ac.jp/els/110003623132.pdf?id=ART0004128721&type=pdf&lang=en&host=cinii&order_no=&ppv_type=0&lang_sw=&no=134 2516782&cp= [gefunden am 2012-06-19]
- Jing Hao ET AL: "First total synthesis of astechrome: novel hydroxamic acid with an indole-pyrazine skeleton", Chemical & Pharmaceutical Bulletin, 1. Januar 1994 (1994-01-01), Seiten 277-279, XP055030353, Gefunden im Internet: URL:http://ci.nii.ac.jp/els/110003630922.pdf?id=ART0004138024&type=pdf&lang=en&host=cinii&order_no=&ppv_type=0&lang_sw=&no=134 2516855&cp= [gefunden am 2012-06-19]
- Arai Kunizo ET AL: "Metabolic products of Aspergillus terreus. VII. Astechrome: an iron-containing metabolite of the strain IFO 6123", Chemical & Pharmaceutical Bulletin, 1. Januar 1981 (1981-01-01), Seiten 1510-1517, XP055030361, Gefunden im Internet: URL:http://ci.nii.ac.jp/els/110003633857.pdf?id=ART0004141421&type=pdf&lang=en&host=cinii&order_no=&ppv_type=0&lang_sw=&no=134 2516922&cp= [gefunden am 2012-06-19]
- Gemma Assante ET AL: "Isolation and structure of red pigments from Aspergillus flavus and related species, grown on a differential medium", Journal of Agricultural and Food Chemistry, 1. Januar 1981 (1981-01-01), Seiten 785-787, XP055030365, Gefunden im Internet: URL:http://pubs.acs.org/doi/pdf/10.1021/jf00106a023 [gefunden am 2012-06-19]
- JING HAO ET AL: "Approach to the synthesis of astechrome", HETEROCYCLES, ELSEVIER SCIENCE PUBLISHERS B.V. AMSTERDAM, NL, Bd. 35, Nr. 2, 1. Januar 1993 (1993-01-01) , Seiten 1279-1287, XP009160458, ISSN: 0385-5414
- SUZUKI, SACHIKO ET AL: "Iron chelated cyclic peptide, ferrichrysin, for oral treatment of iron deficiency: solution properties and efficacy in anemic rats", INTERNATIONAL JOURNAL FOR VITAMIN AND NUTRITION RESEARCH , 77(1), 13-21 CODEN: IJVNAP; ISSN: 0300-9831, 2007, XP009160393,

## Beschreibung

### Einleitung:

Die Erfindung betrifft Eisen(III)-pyrazin-2-ol-1-oxid-Komplexverbindungen und deren sie umfassende pharmazeutische Zusammensetzungen zur Verwendung in der Behandlung und/oder zur Prophylaxe von Eisenmangelerscheinungen und Eisenmangelanämien.

### Hintergrund:

Eisen ist ein essentielles Spurenelement für fast alle Lebewesen und ist dabei insbesondere für das Wachstum und die Blutbildung relevant. Die Balance des Eisenhaushaltes wird dabei primär auf der Ebene der Eisenwiedergewinnung aus Hämoglobin von alternden Erythrozyten und der duodenalen Absorption von nahrungsgebundenem Eisen reguliert. Das freigesetzte Eisen wird über den Darm insbesondere durch spezifische Transportsysteme (DMT-1, Ferroportin, Transferrin, Transferrin Rezeptoren) aufgenommen, in den Blutkreislauf transportiert und hierüber in die entsprechenden Gewebe und Organe weitergeleitet.

Das Element Eisen ist im menschlichen Körper unter anderem für den Sauerstofftransport, die Sauerstoffaufnahme, Zellfunktionen, wie den mitochondrialen Elektronentransport und letztlich für den gesamten Energiestoffwechsel von großer Bedeutung.

Der Körper eines Menschen enthält im Durchschnitt 4 bis 5 g Eisen, wobei dies in Enzymen, in Hämoglobin und Myoglobin, sowie als Depot- oder Reserve-Eisen in Form von Ferritin und Hämosiderin vorliegt.

Etwa die Hälfte dieses Eisens ca. 2 g liegt als Häm Eisen gebunden im Hämoglobin der roten Blutkörperchen vor. Da Erythrozyten nur eine begrenzte Lebensdauer aufweisen (75-150 Tage), müssen ständig neue gebildet und alte eliminiert werden (über 2 Mio. Erythrozyten werden pro Sekunde neu gebildet). Diese hohe Neubildungskapazität wird durch Makrophagen erreicht, indem diese die alternden Erythrozyten phagozytotisch aufnehmen, lysieren und so das enthaltene Eisen für den Eisenhaushalt rezyklieren können. Somit wird die täglich für die Erythropoese benötige Eisenmenge von ca. 25 mg grösstenteils bereit gestellt.

Der tägliche Eisenbedarf eines erwachsenen Menschen beträgt zwischen 0,5 und 1,5 mg pro Tag, bei Kleinkindern sowie bei Frauen in der Schwangerschaft liegt der Eisenbedarf bei 2 bis 5 mg pro Tag. Die täglichen Eisenverluste, z.B. durch Abschilferung von Haut- und Epithelzellen ist gering, erhöhte Eisenverluste treten beispielsweise bei der Menstruationsblutung bei Frauen auf. Generell können Blutverluste den Eisenhaushalt beträchtlich verringern, da pro 2 ml Blut etwa 1 mg Eisen verloren gehen. Der normale tägliche Eisenverlust von ca. 1 mg wird üblicherweise bei einem erwachsenen, gesunden Menschen über die tägliche Nahrungsaufnahme wieder ersetzt. Der Eisenhaushalt wird über Resorption reguliert, wobei die Resorptionsquote des in der Nahrung vorhandenen Eisens zwischen 6 und 12 % beträgt, bei Eisenmangel beträgt die Resorptionsquote bis zu 25 %. Die Resorptionsquote wird vom Organismus in Abhängigkeit vom Eisenbedarf und der Eisenspeichergröße reguliert. Dabei nutzt der menschliche Organismus sowohl zweiwertige als auch dreiwertige Eisenionen. Üblicherweise werden Eisen(III)-Verbindungen bei ausreichend saurem pH-Wert im Magen gelöst und damit für die Resorption verfügbar gemacht. Die Resorption des Eisens findet im oberen Dünndarm durch Mukosazellen statt. Dabei wird dreiwertiges nicht Häm Eisen für die Resorption z.B. durch Ferrireduktase (membranständiges, duodenales Cytochrom b) in der Darmzellmembran zunächst zu Fe(II) reduziert, um dann durch das Transportprotein DMT1 (divalent metal transporter 1) in die Darmzellen transportiert werden zu können. Dagegen gelangt Häm Eisen unverändert über die Zellmembran in die Enterozyten. In den Enterozyten wird Eisen entweder als Depot-Eisen in Ferritin gespeichert oder durch das Transportprotein Ferroportin ins Blut abgegeben. In diesem Vorgang spielt Hepcidin eine zentrale Rolle, da es den wesentlichen Regulationsfaktor der Eisenaufnahme darstellt. Das durch das Ferroportin ins Blut transportierte zweiwertige Eisen wird durch Oxidasen (Ceruloplasmin, Hephaestin) in dreiwertiges Eisen überführt, welches dann mittels Transferrin an die relevanten Stellen im Organismus transportiert wird (s. zum Beispiel: "Balancing acts: molecular control of mammalian iron metabolism". M.W. Hentze, Cell 117, 2004, 285-297).

Der Organismus von Säugetieren kann Eisen nicht aktiv ausscheiden. Der Eisenmetabolismus wird im Wesentlichen über die zelluläre Freisetzung von Eisen aus Makrophagen, Hepatocyten und Enterozyten durch das Hepcidin gesteuert.

Ein verringerter Serum-Eisenspiegel führt in krankhaften Fällen zu einem verringerten Gehalt an Hämoglobin, verringerter Erythrozytenproduktion und damit zu einer Anämie.

Äussere Symptome von Anämien beinhalten Müdigkeit, Blässe sowie verringerte Aufmerksamkeitskapazitäten. Die klinischen Symptome einer Anämie beinhalten niedrige Serum-Eisengehalte (Hypoferrämie), geringe Hämoglobingehalte, geringe Hämatokritlevel sowie eine reduzierte Anzahl an roten Blutkörperchen, reduzierte Retikulozyten und erhöhte Werte an löslichen Transferrinrezeptoren.

Eisenmangelerscheinungen oder Eisenanämien werden durch Eisenzufuhr behandelt. Dabei erfolgt die Substitution mit Eisen entweder auf dem oralen Weg oder durch intravenöse Eisengabe. Außerdem können zur Ankurbelung

der Bildung von roten Blutkörperchen auch Erythropoetin und andere Erythropoese-stimulierende Substanzen bei der Behandlung von Anämien eingesetzt werden.

Anämie kann oft auf Mangelernährung bzw. eisenarme Diäten oder unausgewogene, eisenarme Ernährungsgewohnheiten zurückgeführt werden. Außerdem treten Anämien durch verringerte bzw. schlechte Eisenabsorption beispielsweise aufgrund von Gastrektomien oder von Erkrankungen wie Crohn's-Disease auf. Auch kann ein Eisenmangel infolge eines erhöhten Blutverlustes z.B. durch eine Verletzung, starke Menstruationsblutung oder Blutspende auftreten. Auch ist ein erhöhter Eisenbedarf in der Wachstumsphase Heranwachsender und Kinder sowie von Schwangeren bekannt. Da ein Eisenmangel nicht nur zu einer verringerten Bildung von roten Blutkörperchen, sondern damit auch zu einer schlechten Versorgung des Organismus mit Sauerstoff führt, was zu den oben genannten Symptomen wie Müdigkeit, Blässe und Konzentrationsschwäche auch gerade bei Heranwachsenden zu langfristigen negativen Auswirkungen auf die kognitive Entwicklung führen kann, ist eine gut wirksame und gut verträgliche Therapie von besonderem Interesse.

Durch die Verwendung der erfindungsgemäßen Fe(III)-Komplexverbindungen besteht die Möglichkeit, Eisenmangelerscheinungen und Eisenmangelanämien durch orale Applikation effektiv zu behandeln, ohne das grosse Nebenwirkungspotential der klassischen Präparate, der Fe(II)-Eisensalze, wie $FeSO_4$, hervorgerufen durch Oxidativen Stress, in Kauf zu nehmen. Somit wird eine schlechte Compliance vermieden, die oft Grund für die mangelnde Beseitigung des Eisenmangelzustands darstellt.

**Stand der Technik:**

Aus dem Stand der Technik sind Eisenkomplex-Verbindungen für die Behandlung von Eisenmangelzuständen bekannt.

Ein sehr grosser Anteil dieser Komplex-Verbindungen besteht aus polymeren Strukturen. Hierbei handelt es sich bei den meisten Komplex-Verbindungen um Eisen-Polysaccharid-Komplexverbindungen (WO20081455586, WO2007062546, WO20040437865, US2003236224, EP150085). Gerade aus diesem Bereich sind schon Medikamente auf dem Markt verfügbar (wie Maltofer, Venofer, Ferinject, Dexferrum, Ferumoxytol).

Ein anderer grosser Bestandteil der Gruppe der polymeren Komplexverbindungen sind die Eisen-Peptid-Komplexverbindungen (CN101481404, EP939083, JP02083400).

In der Literatur sind auch Fe-Komplexverbindungen beschrieben, die sich von Makromolekülen, wie Hämoglobin, Chlorophyll, Curcumin und Heparin strukturell herleiten (US474670, CN1687089, Biometals, 2009, 22, 701-710).

Ferner sind in der Literatur auch niedermolekulare Fe-Komplexverbindungen beschrieben. Eine Vielzahl dieser Fe-Komplexverbindungen umfasst Carbonsäuren und Aminosäuren als Liganden. Besonders stehen hier Aspartat (US2009035385) und Citrat (EP308362) als Liganden im Vordergrund. In diesem Zusammenhang werden auch Fe-Komplexverbindungen, die derivatisierte Phenylalanin-Reste als Liganden beinhalten beschrieben (ES2044777).

In der Patent-Literatur sind auch Hydroxypyron- und Hydroxypyridon-Fe-Komplexverbindungen beschrieben (EP159194, EP138420, EP107458, EP0120670). Analog hierzu sind auch die entsprechenden 5-Ringsysteme, die Hydroxyfuranon-Fe-Komplexverbindungen beschrieben (WO2006037449). Insbesondere die Hydroxypyridon-Fe-Komplexverbindungen weisen jedoch eine vergleichsweise geringe Wasserlöslichkeit auf, weshalb sie insbesondere für die orale Verabreichung weniger geeignet sind. Des weiteren weisen die Hydroxypyridon-Fe-Komplexverbindungen eine vergleichsweise geringe Eisenutilisation auf.

INTERNATIONAL JOURNAL FOR VITAMIN AND NUTRITION RESEARCH, 77(1), 13-21 CODEN: IJVNAP; ISSN: 0300-9831, 2007 beschreibt Ferrichrysine zur Verwendung in der Behandlung von Eisenmangelerkrankungen.

Ausserdem sind in der Literatur Eisen-Cyclopentadienyl-Komplexverbindungen beschrieben (GB842637).

Im weiteren wurden 1-Hydroxy-2(1*H*)-pyrazinon (HOPR-H) und 1-Hydroxy-5,6-dimethyl-2(1*H*)-pyrazinon (HOPR-Me) beschrieben (Bull. Chem. Soc. Jpn., 66, 841-841(1993); s. auch "Reviews On Heteroatom Chemistry", Vol. 18, 1998, Seite 87 bis 118 und TETRAHEDRON, ELSEVIER SCIENCE PUBLISHERS, AMSTERDAM, NL, Bd. 51, Nr. 47, 20. November 1995 (1995-11-20), Seiten 12995-13002 derselben Autoren). Für letztere Verbindung wird das UV-vis-Spektrum einer 3:1 molaren Mischung mit Eisen(III) in wässriger Lösung unter verschiedenen pH-Bedingungen gezeigt. Aus den Untersuchungen wird auf die Bildung von 3:1-Komplexen mit Eisen(III) geschlossen. Anders als für die Pyrimidinone wird eine mögliche Struktur der Pyrazinon-Komplexverbindungen nicht gezeigt. Eine Isolierung von Eisen-Komplexverbindungen aus den 3:1-Mischungen in wässriger Lösung erfolgte jedoch nicht. Feste Eisen-Komplexverbindungen wurden demnach nicht isoliert bzw. offenbart. Auch werden die Eisen-Komplexverbindungen nicht als Arzneimittel, insbesondere für die Behandlung von Eisenmangelzuständen vorgeschlagen oder verwendet. Diese Autoren schlagen lediglich die Verwendung sechszähniger 1-Hydroxy-1H-pyrazin-2-on-Verbindungen als Eisenmaskierungsmittel zur Behandlung von Eisenüberladungszuständen, wie Thalassämie, vor (J. Org. Chem. 1997, 62, 3618-3624). Dabei könnte durch eine Verabreichung der Hydroxypyrimidinon- bzw. pyrazin-Verbindungen dem Körper zur Behandlung der Thalassämie Eisen entzogen werden - also nicht Eisen zugeführt werden - wie bei der erfindungsgemäß durchgeführten Behandlung der Eisenmangelanämie durch Verabreichung von Eisen-Komplexverbindungen.

J. Am. Chem. Soc. 1985, 107, 6540-6546 beschreibt vierzähnige 1-Hydroxy-1H-pyridin-2-on-Verbindungen als Liganden und eine zweikernige Eisenkomplexverbindung damit. Die Möglichkeit den Liganden zur Eisenmaskierung zu verwenden wird ebenfalls erwähnt. Ähnlich offenbart Inorganica Chimica Acta, 135 (1987) 145-150 die Verwendung von 1-Hydroxy-1H-pyridin-2-onen als Mittel zur Eisenmaskierung.

NATURAL PRODUCT COMMUNICATIONS; 2011, 6(8),1137-1140 und Journal of Agricultural and Food Chemistry, 1. Januar 1981 (1981 -01-01), Seiten 785-787 beschreiben Eisen(III)-Komplexe der Neoaspergillinsäure, wobei erstgenannte Publikation auch allgemein deren antibakterielle Aktivität erwähnt. Chemical & Pharmaceutical Bulletin, 1. Januar 1978 (1978-01-01), Seiten 1320-1322 beschreibt strukturell ähnliche Eisenkomplex-Verbindungen. Die Verwendung derartiger Eisen-Komplexe als Arzneimittel, insbesondere in der Behandlung von Eisenmangelanämie wird nicht erwähnt.

Chemical & Pharmaceutical Bulletin, 1. Januar 1994 (1994-01-01), Seiten 277-279, Chemical & Pharmaceutical Bulletin, 1. Januar 1981 (1981-01-01), Seiten 1510-1517 und HETEROCYCLES, ELSEVIER SCIENCE PUBLISHERS B.V. AMSTERDAM, NL, Bd. 35, Nr. 2, 1. Januar 1993 (1993-01-01), Seiten 1279-1287 beschreiben Astechrome und deren Isolierung als Stoffwechselprodukt aus Aspergillus-Stämmen. Eine Verwendung solcher Eisen-Komplexe zur Verwendung als Arzneimittel, insbesondere in der Behandlung von Eisenmangelanämien wird nicht beschrieben.

Ein anderer wichtiger Bestandteil für die Behandlung von Eisenmangelerscheinungen und Eisenmangelanämien stellen die Eisensalze (z.B. Eisen(II)sulfat, Eisen(II)fumarat, Eisen(III)chlorid, Eisen(II)aspartat, Eisen(II)succinat) dar.

Ein grosses Problem dieser Eisensalze stellt ihre teilweise hohe Unverträglichkeit (bis zu 50%) in Form von Übelkeit, Erbrechen, Durchfall aber auch Obstipation und Krämpfe dar. Ausserdem kommt es bei der Verwendung von diesen Eisen(II)-salzen zum Auftreten freier Fe(II)Ionen, die die Bildung (u.a. Fenton Reaktion) von reaktiven Sauerstoff Spezies (ROS) katalysieren. Durch diese ROS kommt es zur Beschädigung von DNA, Lipiden, Proteinen und Kohlenhydraten, was weitreichende Auswirkungen auf Zellen, Gewebe und Organe hat. Diese Problematik ist bekannt und wird in der Literatur weitgehend als Ursache für die hohe Unverträglichkeit angesehen und als Oxidativer Stress bezeichnet.

Eisen (III)-pyrazin-2-ol-1-oxid Komplexverbindungen sind im Stand der Technik bekannt: das Dokument Natural Product Communications 6(8), 2011, 1137-1140 offenbart die antibaktierrellle Aktivität von Ferrineoaspergillin (Verbindung 2) und das Dokument J. Org. Chem, 62, 1997, 3618-3624 offenbart die Entfernung von Eisen(III) mittels einer Eisen(III)-Komplexverbindung (Figure 2). Eisen(III)-1-Hydroxy-1H-pyrazin-2-on- bzw. Pyrazin-2-ol 1-oxid-Komplexverbindungen sind im Stand der Technik somit zur Verwendung in der Behandlung und/oder zur Prophylaxe von Eisenmangelerscheinungen und Eisenmangelanämien nicht beschrieben worden.

**Aufgabenstellung:**

Die Aufgabe der vorliegenden Erfindung bestand darin, neue therapeutisch wirksame Verbindungen zu entwickeln, die für eine effektive Therapie zur bevorzugt oralen Behandlung von Eisenmangelerscheinungen und Eisenmangelanämien eingesetzt werden können. Dabei sollten diese Eisenkomplexe gleichzeitig deutlich weniger Nebenwirkungen aufzeigen, als die klassisch verwendeten Fe(II)-Salze. Ausserdem sollten diese Eisenkomplexe im Unterschied zu den bekannten polymeren Eisenkomplexverbindungen möglichst eine definierte Struktur (Stöchiometrie) aufweisen und durch einfache Synthesewege darstellbar sein. Des weiteren sollten die Verbindungen bei oraler Verabreichung zu einer hohen Utilisationsrate des Eisens führen, was durch eine gute Wasserlöslichkeit unterstützt wird. Schließlich sollten die Eisenkomplex-Verbindungen über eine möglichst geringe Toxizität verfügen und somit in möglichst hohen Dosierungen verabreichbar sein. Dieses Ziel wurde durch die Entwicklung neuartiger Fe(III)-Komplexverbindungen erreicht.

Weiterhin sollten die neuen Eisenkomplexe so beschaffen sein, das sie direkt über die Membran in die Darmzellen aufgenommen werden, um somit ihr komplexgebundenes Eisen direkt an das Ferritin oder an das Transferrin abzugeben oder direkt als intakter Komplex in die Blutbahn zu gelangen. Die neuen Verbindungen sollten aufgrund ihrer Eigenschaften praktisch nicht zum Auftreten von hohen Konzentrationen an freien Eisenionen führen. Denn gerade durch freie Eisenionen kommt es zum Auftreten von ROS, die letztlich für die auftretenden Nebenwirkungen verantwortlich sind.

Um diese Anforderungen erreichen zu können, entwickelten die Erfinder neue Fe(III)-Komplexverbindungen mit nicht zu grossem Molekulargewicht, mittlerer Lipophilie und optimaler Komplexstabilität.

**Beschreibung der Erfindung:**

Die Erfinder fanden überraschend, dass Fe(III)-Komplexverbindungen mit Pyrazin-2-ol 1-oxid-Liganden sich für die oben beschriebenen Anforderungen besonders eignen. Es konnte gezeigt werden, dass diese Fe-Komplexverbindungen eine hohe Eisenaufnahme zeigen, wodurch ein schneller Therapieerfolg bei der Behandlung der Eisenmangelanämie erreicht werden kann. Gerade im Vergleich mit Eisensalzen zeigen die erfindungsgemäßen Komplexverbindungen eine schnellere und höhere Utilisation. Ausserdem weisen diese neuen Systeme deutlich geringere Nebenwirkungen als die klassisch verwendeten Eisensalze auf, da es hier nicht in nennenswertem Ausmaß zum Auftreten von freien Eisenionen kommt. Die erfindungsgemäßen Komplexverbindungen zeigen nahezu keinen Oxidativen Stress, da es nicht zur Bildung von freien Radikalen kommt. Somit treten bei diesen Komplexverbindungen deutlich weniger Nebenwirkungen als bei den aus dem Stand der Technik bekannten Fe Salzen auf. Die Komplexverbindungen zeigen in unterschiedlichen pH-Wert-Bereichen gute Stabilitäten und vergleichsweise gute Löslichkeiten. Des Weiteren verfügen die Eisenkomplex-Verbindungen über eine sehr geringe Toxizität und können somit in hohen Dosierungen ohne Nebenwirkungen verabreicht werden. Die Komplexverbindungen lassen sich gut herstellen und eignen sich optimal zur Formulierung von Arzneimitteln, insbesondere zur oralen Verabreichung.

Gegenstand der Erfindung sind somit Eisen(III)-pyrazin-2-ol 1-oxid-Komplexverbindungen oder deren pharmazeutisch verträgliche Salze zur Verwendung in der Behandlung und Prophylaxe von Eisenmangelerscheinungen und Eisenmangelanämien sowie der damit einhergehenden Symptome.

Die erfindungsgemäß verwendeten Eisen(III)-pyrazin-2-ol 1-oxid-Komplexverbindungen schließen insbesondere solche Verbindungen ein, die folgendes Strukturelement aufweisen:

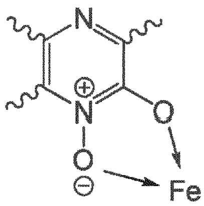

worin



jeweils ein die freie Valenz absättigender Substituent des Liganden ist und die Pfeile jeweils koordinative Bindungen zum Eisenatom darstellen. Die Begriffe

- "Pyrazin-2-ol 1-oxid",
- "Pyrazin-2-ol 1-oxid-Verbindungen" oder
- "Pyrazin-2-ol 1-oxid-"-Liganden

schließen somit erfindungsgemäß sowohl die entsprechenden Hydroxy-Ausgangverbindungen

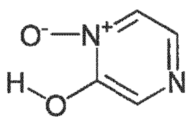

als auch die entsprechenden deprotonierten Liganden

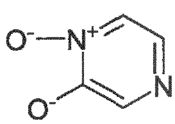 bzw. 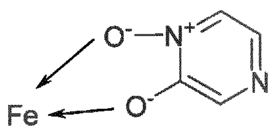 ,

ein, welche in den entsprechenden Eisen(III)-Komplexverbindungen vorliegen.

Desweiteren schließen die genannten Begriffe erfindungsgemäß nicht nur den entsprechenden Grundkörper:

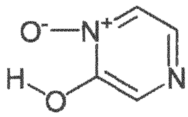

bzw. die durch Deprotonierung aus der zugrundeliegenden Hydroxyverbindung

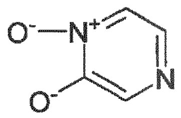

hervorgegangene Ligand-Verbindung ein, sondern auch deren am Pyrazinring substituierte Vertreter, welche aus dem Austausch eines oder mehrerer Wasserstoffatome am Pyrazinring gegen andere Substituenten resultieren. Die genannten Begriffe bezeichnen somit erfindungsgemäß die Gesamtheit der Klasse der "Pyrazin-2-ol 1-oxid"-Verbindungen bzw. der deprotonierten Liganden, einschließlich der am Pyrazinring substituierten Vertreter.

Ein (deprotonierter) Pyrazin-2-ol 1-oxid-Ligand wie er erfindungsgemäß verwendet wird, trägt somit formal eine negative Ladung. Das bedeutet, dass bei drei Liganden je Eisenatom das Eisenatom formal die Oxidationsstufe +3 besitzt. Es ist für den Fachmann klar, dass die gezeigten Formeln nur eine mögliche mesomere Grenzformel darstellen, und dass mehrere mesomere Grenzformeln existieren bzw. eine Delokalisierung der Elektronen im Liganden bzw. der Eisen-Komplexverbindung vorliegt, wie nachfolgend noch schematisch gezeigt wird.

In den erfindungsgemäßen Eisen(III)-pyrazin-2-ol 1-oxid-Komplexverbindungen beträgt die Koordinationszahl der Eisenatome im Allgemeinen sechs (6), wobei im Allgemeinen eine oktaedrische Anordnung der koordinierenden Atome vorliegt.

Erfindungsgemäß sind weiterhin ein- oder mehrkernige Eisen(III)-pyrazin-2-ol 1-oxid-Komplexverbindungen umfasst, in denen ein oder mehrere (wie 2, 3 oder 4) Eisenatome vorhanden sind.

Im allgemeinen können 1-4 Eisenatome und 2-10 Liganden in den Eisen(III)-pyrazin-2-ol 1-oxid-Komplexverbindungen vorliegen. Bevorzugt sind jedoch einkernige Eisen(III)-pyrazin-2-ol 1-oxid-Komplexverbindungen mit mindestens einem bevorzugt 3, bevorzugt zweizähnigen Pyrazin-2-ol 1-oxid-Liganden. Bevorzugt sind jedoch einkernige Eisen(III)-pyrazin-2-ol 1-oxid-Komplexverbindungen, in denen ein (1) zentrales Eisenatom und drei (3) Pyrazin-2-ol 1-oxid-Liganden vorliegen.

Die erfindungsgemäßen Eisen(III)-pyrazin-2-ol 1-oxid-Komplexverbindungen liegen im Allgemeinen in neutraler Form vor. Es sind jedoch auch salzartige Eisen(III)-pyrazin-2-ol 1-oxid-Komplexverbindungen eingeschlossen, in denen der Komplex eine positive oder negative Ladung aufweist, die insbesondere durch pharmakologisch verträgliche, im wesentlichen nicht-koordinierende Anionen (wie insbesondere Halogenide, wie Chlorid) oder Kationen (wie insbesondere Alkali- oder Erdalkalimetallionen) ausgeglichen wird.

Die erfindungsgemäßen Eisen(III)-pyrazin-2-ol 1-oxid-Komplexverbindungen schließen insbesondere solche Komplexverbindungen ein, die mindestens einen, bevorzugt zweizähnigen Pyrazin-2-ol 1-oxid-Liganden der Formel

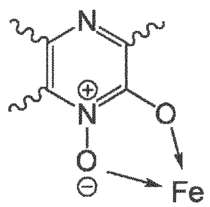

aufweisen, worin  jeweils ein die freie Valenz absättigender Substituent des Liganden ist, der, wie vorstehend gezeigt, an ein oder auch an zwei verschiedene Eisenatome im Sinne einer Verbrückung gebunden sein kann.

Bevorzugt sind Eisen(III)-pyrazin-2-ol 1-oxid-Komplexverbindungen, die ausschließlich, bevorzugt zweizähnige Pyrazin-2-ol 1-oxid-Liganden aufweisen, welche gleich oder verschieden sein können. Besonders bevorzugt sind weiterhin Eisen(III)-pyrazin-2-ol 1-oxid-Komplexverbindungen, die ausschließlich die gleichen Pyrazin-2-ol 1-oxid-Liganden aufweisen, und ganz besonders bevorzugt sind Tris(pyrazin-2-ol 1-oxid)-eisen(III)-Verbindungen.

Bevorzugt liegt das Molekulargewicht der erfindungsgemäßen Eisen(III)-pyrazin-2-ol 1-oxid-Komplexverbindungen bei weniger als 1000 g/mol, noch bevorzugter bei weniger als 800 g/mol (jeweils bestimmt aus der Strukturformel).

In einer besonders bevorzugten Ausführungsform enthalten die erfindungsgemäßen Eisen(III)-Komplexverbindungen mindestens einen, bevorzugt drei gleiche oder verschiedene, bevorzugt gleiche Liganden der Formel (I):

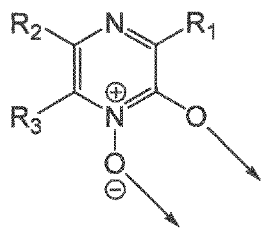

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- gegebenenfalls substituiertem Alkyl,
- Halogen,
- gegebenenfalls substituiertem Alkoxy,
- gegebenenfalls substituiertem Aryl,
- gegebenenfalls substituiertem Alkoxycarbonyl, und
- gegebenenfalls substituiertem Aminocarbonyl ist, oder

$R_2$ und $R_3$ gemeinsam mit den Kohlenstoffatomen, an die sie gebunden sind, einen gegebenenfalls substituierten 5- oder 6-gliedrigen gesättigten oder ungesättigten Ring bilden, der gegebenenfalls ein oder mehrere Heteroatome aufweisen kann, oder pharmazeutisch verträgliche Salze davon.

Die vorstehend erwähnte Ringbildung der Substituenten $R_2$ und $R_3$ ist schematisch in der folgenden Formel gezeigt:

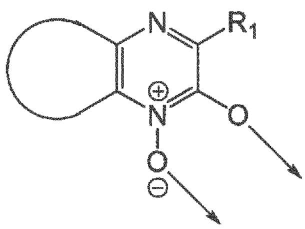

.

Darin kann $R_1$ eine der genannten Bedeutungen aufweisen.

In einer bevorzugten Ausführungsform der Erfindung betrifft diese Eisen(III)-Komplexverbindungen enthaltend mindestens einen Liganden der Formel (I):

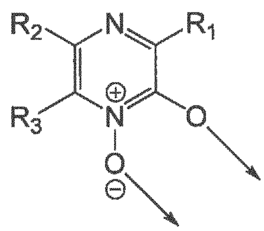

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff, und
- gegebenenfalls substituiertem Alkyl.

Gegebenenfalls substituiertes Alkyl schließt im Rahmen der gesamten Erfindung insbesondere für die Substituenten $R_1$ bis $R_3$ bevorzugt ein:

Geradkettiges oder verzweigtes Alkyl mit 1 bis 8, bevorzugt 1 bis 6, Kohlenstoffatomen, Cycloalkyl mit 3 bis 8, bevorzugt 5 oder 6 Kohlenstoffatomen, oder Alkyl mit 1 bis 4 Kohlenstoffatomen, welches mit Cycloalkyl substituiert ist, wobei diese Alkylgruppen gegebenenfalls substituiert sein können.

Die genannten Alkylgruppen können unsubstituiert oder substituiert, bevorzugt mit 1 bis 3 Substituenten, sein. Diese Substituenten an den Alkylgruppen werden bevorzugt aus der Gruppe ausgewählt, die besteht aus: Hydroxy, gegebenenfalls substituiertem Aryl, insbesondere wie nachstehend definiert, gegebenenfalls substituiertem Heteroaryl, insbesondere wie nachstehend definiert, gegebenenfalls substituiertem Alkoxy insbesondere wie nachstehend definiert, gegebenenfalls substituiertem Alkoxycarbonyl, insbesondere wie nachstehend definiert, gegebenenfalls substituiertem Acyl, insbesondere wie nachstehend definiert, Halogen, insbesondere wie nachstehend definiert, gegebenenfalls substituierten Amino, insbesondere wie nachstehend definiert, gegebenenfalls substituierten Aminocarbonyl, insbesondere wie nachstehend definiert, und Cyano.

Halogen schließt hier und im Rahmen der vorliegenden Erfindung Fluor, Chlor, Brom und Iod, bevorzugt Fluor oder Chlor, ein.

In den vorstehend definierten Alkylgruppen können des weiteren gegebenenfalls ein oder mehrere, bevorzugter 1 bis 3 Kohlenstoffatome durch heteroanaloge Gruppen, die Stickstoff, Sauerstoff oder Schwefel enthalten, ersetzt sein. Dies bedeutet insbesondere, dass beispielsweise eine oder mehrere, bevorzugt 1 bis 3, noch bevorzugter eine (1) Methylengruppe ($-CH_2-$) in den Alkylresten durch -NH-, $-NR_4-$, -O- oder -S- ersetzt sein können, worin $R_4$ gegebenenfalls substituiertes Alkyl, wie vorstehend definiert, bevorzugt gegebenenfalls mit 1 bis 3 Substituenten, wie Fluor, Chlor, Hydroxy oder Alkoxy, substituiertes C1-C6 Alkyl, wie Methyl oder Ethyl ist.

Beispiele von Alkylresten mit 1 bis 8 Kohlenstoffatomen schließen ein: eine Methylgruppe, eine Ethylgruppe, eine n-Propylgruppe, eine i-Propylgruppe, eine n-Butylgruppe, eine i-Butylgruppe, eine sec-Butylgruppe, eine t-Butylgruppe, eine n-Pentylgruppe, eine i-Pentylgruppe, eine sec-Pentylgruppe, eine t-Pentylgruppe, eine 2-Methylbutylgruppe, eine n-Hexylgruppe, eine 1-Methylpentylgruppe, eine 2-Methylpentylgruppe, eine 3-Methylpentylgruppe, eine 4-Methylpentylgruppe, eine 1-Ethylbutylgruppe, eine 2-Ethylbutylgruppe, eine 3-Ethylbutylgruppe, eine 1,1-Dimethylbutylgruppe, eine 2,2-Dimethylbutylgruppe, eine 3,3-Dimethylbutylgruppe, eine 1-Ethyl-1-methylpropylgruppe, eine n-Heptylgruppe, eine 1-Methylhexylgruppe, eine 2-Methylhexylgruppe, eine 3-Methylhexylgruppe, eine 4-Methylhexylgruppe, eine 5-Methylhexylgruppe, eine 1-Ethylpentylgruppe, eine 2-Ethylpentylgruppe, eine 3-Ethylpentylgruppe, eine 4-Ethylpentylgruppe, eine 1,1-Dimethylpentylgruppe, eine 2,2-Dimethylpentylgruppe, eine 3,3-Dimethylpentylgruppe, eine 4,4-Dimethylpentylgruppe, eine 1-Propylbutylgruppe, eine n-Octylgruppe, eine 1-Methylheptylgruppe, eine 2-Methylheptylgruppe, eine 3-Methylheptylgruppe, eine 4-Methylheptylgruppe, eine 5-Methylheptylgruppe, eine 6-Methylheptylgruppe, eine 1-Ethylhexylgruppe, eine 2-Ethylhexylgruppe, eine 3-Ethylhexylgruppe, eine 4-Ethylhexylgruppe, eine 5-Ethylhexylgruppe, eine 1,1-Dimethylhexylgruppe, eine 2,2-Dimethylhexylgruppe, eine 3,3-Dimethylhexylgruppe, eine 4,4-Dimethylhexylgruppe, eine 5,5-Dimethylhexylgruppe, eine 1-Propylpentylgruppe, eine 2-Propylpentylgruppe, usw. Bevorzugt sind solche mit 1 bis 6 Kohlenstoffatomen. Am meisten bevorzugt sind Methyl, Ethyl, n-Propyl, Isopropyl, sek.-Butyl und n-Butyl.

Beispiele von Alkylgruppen, die durch Austausch mit einer oder mehreren heteroanalogen Gruppe, wie -O-, -S-, -NH- oder $-N(R_4)-$ hervorgehen, sind bevorzugt solche, in denen eine oder mehrere Methylengruppen ($-CH_2-$) durch -O- unter Bildung einer Ethergruppe ersetzt sind, wie Methoxymethyl, Ethoxymethyl, 2-Methoxyethyl usw. Daher schließt die Definition von Alkyl auch beispielsweise Alkoxyalkylgruppen, wie nachstehend definiert, ein, welche durch Austausch einer Methylengruppe durch -O- aus den genannten Alkylgruppen hervorgehen. Sind erfindungsgemäß darüber hinaus Alkoxygruppen als Substituenten von Alkyl zugelassen, können auch auf diese Weise mehrere Ethergruppen gebildet werden (wie zum Beispiel eine $-CH_2-O-CH_2-OCH_3$-Gruppe). Erfindungsgemäß sind somit auch Polyethergruppen von der Definition von Alkyl umfasst.

Cycloalkylreste mit 3 bis 8 Kohlenstoffatomen schließen bevorzugt ein: eine Cyclopropylgruppe, eine Cyclobutylgruppe, eine Cyclopentylgruppe, eine Cyclohexylgruppe, eine Cycloheptylgruppe, eine Cyclooctylgruppe usw. ein. Bevorzugt sind eine Cyclopropylgruppe, eine Cyclobutylgruppe, eine Cyclopentylgruppe und eine Cyclohexylgruppe. Die Cycloalkylreste können gegebenenfalls substituiert sein, bevorzugt mit 1 bis 2 Substituenten, wie Hydroxy oder C1-C6-Alkoxycarbonyl.

Die Definition der gegebenenfalls substituierten Alkylgruppen schließt auch Alkylgruppen ein, welche durch vorstehend definierte Cycloalkylgruppen substituiert sind, wie beispielsweise Cyclopropylmethyl, Cyclobutylmethyl, Cyclopentylmethyl oder Cyclohexylmethyl.

Heterocyclische Alkylreste sind erfindungsgemäß insbesondere solche, die durch Austausch von Methylen durch Heteroanaloge Gruppen aus Cycloalkyl gebildet werden, und schließen beispielsweise gesättigte 5- oder 6-gliedrige heterocyclische Reste ein, welche über ein Kohlenstoffatom oder ein Stickstoffatom angebunden sein können, und welche bevorzugt 1 bis 3, bevorzugt 2 Heteroatome wie insbesondere O, N aufweisen können, wie Tetrahydrofuryl, Azetidin-1-yl, substituiertes Azetidinyl, wie 3-Hydroxyazetidin-1-yl, Pyrrolidinyl, wie Pyrrolidin-1-yl, substituiertes Pyrrolidinyl, wie 3-Hydroxypyrrolidin-1-yl, 2-Hydroxypyrrolidin-1-yl, 2-Methoxycarbonylpyrrolidin-1-yl, 2-Ethoxycarbonylpyrrolidin-1-yl, 2-Methoxypyrrolidin-1-yl, 2-Ethoxypyrrolidin-1-yl, 3-Methoxycarbonylpyrrolidin-1-yl, 3-Ethoxycarbonylpyrrolidin-1-yl, 3-Methoxypyrrolidin-1-yl, 3-Ethoxypyrrolidin-1-yl, Piperidinyl, wie Piperidin-1-yl, Piperidin-4-yl, substituiertes Piperidinyl, wie 4-Methyl-1-piperidyl,4-Hydroxy-1-piperidyl, 4-Methoxy-1-piperidyl, 4-Ethoxy-1-piperidyl, 4-Methoxycarbonyl-1-piperidyl, 4-Ethoxycarbonyl-1-piperidyl, 4-Carboxy-1-piperidyl, 4-Acetyl-1-piperidyl,4-Formyl-1-piperidyl, 1-Methyl-4-piperidyl, 4-Hydroxy-2,2,6,6-tetramethyl-1-piperidyl, 4-(Dimethylamino)-1-piperidyl, 4-(Diethylamino)-1-piperidyl, 4-Amino-1-piperidyl, 2-(Hydroxymethyl)-1-piperidyl, 3-(Hydroxymethyl)-1-piperidyl, 4-(Hydroxymethyl)-1-piperidyl, 2-Hydroxy-1-piperidyl, 3-Hydroxy-1-piperidyl, 4-Hydroxy-1-piperidyl, Morpholin-4-yl, substituiertes Morpholinyl, wie 2,6-Dimethylmorpholin-4-yl, Piperazinyl, wie Piperazin-1-yl, substituiertes Piperazinyl, wie 4-Methylpiperazin-1-yl, 4-Ethylpiperazin-1-yl, 4-Ethoxycarbonylpiperazin-1-yl, 4-Methoxycarbonylpiperazin-1-yl, oder Tetrahydropyranyl, wie Tetrahydropyran-4-yl, und welche gegebenenfalls mit aromatischen Ringen kondensiert sein können, und welche gegebenenfalls substituiert sein können, wie mit 1 bis 2 Substituenten, wie Hydroxy, Halogen, C1-C6-Alkyl etc. Die Definition der gegebenenfalls substituierten Alkylgruppen schließt somit auch Alkylgruppen ein, welche durch vorstehend definierte heterocyclische Gruppen substituiert sind, wie beispielsweise 3-(1-Piperidyl)propyl, 3-Pyrrolidin-1-ylpropyl, 3-Morpholinopropyl, 2-Morpholinoethyl, 2-Tetrahydropyran-4-ylethyl, 3-Tetrahydropyran-4-ylpropyl, 3-(Azetidin-1-yl)propyl etc..

Beispiele eines mit Halogen substituierten linearen oder verzweigten Alkylrestes mit 1 bis 8 bevorzugt 1 bis 6 Kohlenstoffatomen schließen insbesondere ein: eine Fluormethylgruppe, eine Difluormethylgruppe, eine Trifluormethylgruppe, eine Chlormethylgruppe, eine Dichlormethylgruppe, eine Trichlormethylgruppe, eine Brommethylgruppe, eine Dibrommethylgruppe, eine Tribrommethylgruppe, eine 1-Fluorethylgruppe, eine 1-Chlorethylgruppe, eine 1-Bromethylgruppe, eine 2-Fluorethylgruppe, eine 2-Chlorethylgruppe, eine 2-Bromethylgruppe, eine 1,2-Difluorethylgruppe, eine 1,2-Dichlorethylgruppe, eine 1,2-Dibromethylgruppe, eine 2,2,2-Trifluorethylgruppe, eine Heptafluorethylgruppe, eine 1-Fluorpropylgruppe, eine 1-Chlorpropylgruppe, eine 1-Brompropylgruppe, eine 2-Fluorpropylgruppe, eine 2-Chlorpropylgruppe, eine 2-Brompropylgruppe, eine 3-Fluorpropylgruppe, eine 3-Chlorpropylgruppe, eine 3-Brompropylgruppe, eine 1,2-Difluorpropylgruppe, eine 1,2-Dichlorpropylgruppe, eine 1,2-Dibrompropylgruppe, eine 2,3-Difluorpropylgruppe, eine 2,3-Dichlorpropylgruppe, eine 2,3-Dibrompropylgruppe, eine 3,3,3-Trifluorpropylgruppe, eine 2,2,3,3,3-Pentafluorpropylgruppe, eine 2-Fluorbutylgruppe, eine 2-Chlorbutylgruppe, eine 2-Brombutylgruppe, eine 4-Fluorbutylgruppe, eine 4-Chlorbutylgruppe, eine 4-Brombutylgruppe, eine 4,4,4-Trifluorbutylgruppe, eine 2,2,3,3,4,4,4-Heptafluorbutylgruppe, eine Perfluorbutylgruppe, eine 2-Fluorpentylgruppe, eine 2-Chlorpentylgruppe, eine 2-Brompentylgruppe, eine 5-Fluorpentylgruppe, eine 5-Chlorpentylgruppe, eine 5-Brompentylgruppe, eine Perfluorpentylgruppe, eine 2-Fluorhexylgruppe, eine 2-Chlorhexylgruppe, eine 2-Bromhexylgruppe, eine 6-Fluorhexylgruppe, eine 6-Chlorhexylgruppe, eine 6-Bromhexylgruppe, eine Perfluorhexylgruppe, eine 2-Fluorheptylgruppe, eine 2-Chlorheptylgruppe, eine 2-Bromheptylgruppe, eine 7-Fluorheptylgruppe, eine 7-Chlorheptylgruppe, eine 7-Bromheptylgruppe, eine Perfluorheptylgruppe, usw.

Beispiele eines mit Hydroxy substituierten Alkylrestes schließen die oben genannten Alkylreste ein, die 1 bis 3 Hydroxylreste aufweisen, wie zum Beispiel Hydroxymethyl, 2-Hydroxyethyl, 3-Hydroxypropyl, 4-Hydroxybutyl, 5-Hydroxypentyl, 6-Hydroxyhexyl.

Gegebenenfalls substituiertes Aryl schließt erfindungsgemäß bevorzugt aromatische Kohlenwasserstoffreste mit 6 bis 14 Kohlenstoffatomen (ohne Heteroatome im aromatischen Ringsystem) ein, wie beispielweise: Phenyl, Naphthyl, Phenanthrenyl und Anthracenyl. Die genannten aromatischen Reste können unsubstituiert oder substituiert sein. Im Falle der Substitution weisen sie bevorzugt einen oder mehrere, bevorzugt einen (1) oder zwei (2) Substituenten auf, wie insbesondere Halogen, Hydroxy, Alkyl, Alkoxy, jeweils wie vorstehend oder nachstehend erläutert. Bevorzugter aromatischer Rest ist Phenyl. Bevorzugtes mit einer aromatischen Gruppe substituiertes Alkyl (Arylalkyl) ist Benzyl.

Gegebenenfalls substituiertes Aryl schließt erfindungsgemäß weiterhin gegebenenfalls substituiertes Heteroaryl also heteroaromatische Reste ein, wie beispielsweise: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl. 5- oder 6-gliedrige aromatische Heterocyclen wie z.B. Pyridyl, Pyridyl-N-oxid, Pyrimidyl, Pyridazinyl, Furyl und Thienyl sind bevorzugt. Die genannten heteroaromatischen Reste können unsubstituiert oder substituiert sein. Im Falle der Substitution weisen sie bevorzugt einen oder mehrere, bevorzugt einen (1) oder (2) Substituenten auf, insbesondere Halogen, Hydroxy, Alkyl, Alkoxy, jeweils wie vorstehend oder nachstehend erläutert. Bevorzugte Beispiele einer mit einer heteroaromatischen Gruppe substituiertes Alkyl (Hetarylalkyl) sind Methyl, Ethyl oder Propyl jeweils substituiert mit einer heteroaromatischen Gruppe, wie Thienylmethyl, Pyridylmethyl etc.

Gegebenenfalls substituiertes Alkoxy (RO-) leitet sich formal durch Hinzufügen eines Sauerstoffatom zu einem der vorstehend erwähnten gegebenenfalls substituierten Alkylreste ab, und schließt erfindungsgemäß beispielsweise lineare oder verzweigte Alkoxyreste bevorzugt mit bis zu 6 Kohlenstoffatomen ein, wie eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, eine n-Pentyloxygruppe, eine i-Pentyloxygruppe, eine sec-Pentyloxygruppe, eine t-Pentyloxygruppe, eine 2-Methylbutoxygrupe, eine n-Hexyloxygruppe, eine i-Hexyloxygruppe, eine t-Hexyloxygruppe, eine sec-Hexyloxygruppe, eine 2-Methylpentyloxygruppe, eine 3-Methylpentyloxygruppe, eine 1-Ethylbutyloxygruppe, eine 2-Ethylbutyloxygruppe, eine 1,1-Dimethylbutyloxygruppe, eine 2,2-Dimethylbutyloxygruppe, eine 3,3-Dimethylbutyloxygruppe, eine 1-Ethyl-1-methylpropyloxygruppe, usw. ein. Bevorzugt sind eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, usw. Die Alkoxygruppen können gegebenenfalls substituiert sein, wie beispielsweise mit den für Alkyl genannten möglichen Substituenten.

Bevorzugtes Alkoxy ist Methoxy, Ethoxy, n-Propoxy, n-Butoxy etc..

Gegebenenfalls substituierte Alkoxycarbonyl (RO-CO-)-Gruppen leiten sich aus den vorstehend definierten Alkylgruppen formal durch Hinzufügen eines -O-C(O)-Restes ab unter Bildung eines gegebenenfalls substituierten Alkyloxycarbonyl-Restes ab. Insoweit kann auf die Definition der vorstehend beschriebenen Alkylgruppen verwiesen werden. Alternativ leiten sich gegebenenfalls substituierte Alkoxycarbonyl (RO-CO-)-Gruppen aus den vorstehend genannten Alkoxygruppen entsprechend formal durch Hinzufügen einer Carbonylgruppe ab. Bevorzugte Alkoxycarbonyl-Gruppen weisen bis zu 6 Kohlenstoffatome auf und schließen beispielsweise ein: Methoxycarbonyl, Ethoxycarbonyl, n-Propoxycarbonyl, n-Butoxycarbonyl, tert.-Butoxycarbonyl etc., welche sämtlich wie vorstehend definierte Alkylgruppen substituiert sein können.

Gegebenenfalls substituiertes Aminocarbonyl leitet sich im Rahmen der Erfindung formal von gegebenenfalls substituiertem Amino durch Hinzufügen eines Carbonylrestes ab ($(R)_2N-C(=O)-$). Dabei schließt gegebenenfalls substituiertes Amino erfindungsgemäß bevorzugt ein: Amino ($-NH_2$), gegebenenfalls substituiertes Mono- oder Dialkylamino (RHN-, $(R)_2N-$) wobei hinsichtlich der Definition von gegebenenfalls substituiertem Alkyl auf vorstehende Definition verwiesen werden kann. Ferner sind gegebenenfalls substituierte Mono- oder Diarylamino-Reste eingeschlossen oder gemischte gegebenenfalls substituierte Alkylarylamino-Reste, wobei hinsichtlich der Definition von gegebenenfalls substituiertem Alkyl oder Aryl auf vorstehende Definition verwiesen werden kann. Derartige Gruppen schließen beispielsweise Methylamino, Dimethylamino, Ethylamino, Hydroxyethylamino, wie 2-Hydroxyethylamino, Diethylamino, Phenylamino, Methylphenylamino etc. ein. Gegebenenfalls substituiertes Amino schließt weiterhin gegebenenfalls substituiertes cyclisches Amino ein, wie gegebenenfalls substituiertes 5 oder 6-gliedriges cyclisches Amino, dass gegebenenfalls weitere Heteroatome wie beispielsweise N, O, S, bevorzugt O enthalten kann. Beispiele von solchen cyclischen Aminogruppen schließen ein die vorstehend genannten stickstoffenthaltenden heterocyclischen Gruppen, die über ein Stickstoffatom angebunden sind, wie Piperidin-1-yl, 4-Hydroxy-piperidin-1-yl, 2-(Methoxycarbonyl)pyrrolidin-1-yl, Pyrrolidin-1-yl, Morpholin-4-yl, etc.

Beispiele von gegebenenfalls substituiertem Aminocarbonyl schließen demnach ein: Carbamoyl ($H_2NC(=O)-$), gegebenenfalls substituiertes Mono- oder Dialkylaminocarbonyl ($RHNC(=O)-$, $(R)_2NC(=O)-$) wobei hinsichtlich der Definition von gegebenenfalls substituiertem Alkyl auf vorstehende Definition verwiesen werden kann. Ferner sind gegebenenfalls substituierte Mono- oder Diarylaminocarbonyl-Reste eingeschlossen oder gemischte gegebenenfalls substituierte Alkylarylaminocarbonyl-Reste, wobei hinsichtlich der Definition von gegebenenfalls substituiertem Alkyl oder Aryl auf vorstehende Definition verwiesen werden kann. Bevorzugte substituierte Aminocarbonyl-Gruppen weisen bis zu 14 Kohlenstoffatome auf. Derartige Gruppen schließen beispielsweise Methylaminocarbonyl, Dimethylaminocarbonyl, Ethylaminocarbonyl, Diethylaminocarbonyl, Phenylaminocarbonyl, Diphenylaminocarbonyl, Methylphenylaminocarbonyl etc. ein.

Beispiele der vorstehend erwähnten Ringbildung der Substituenten $R_2$ und $R_3$ wie schematisch in der folgenden Formel gezeigt:

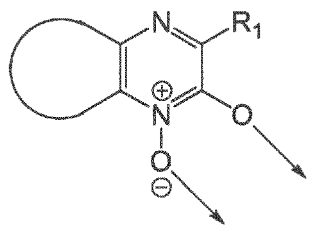

(worin $R_1$ die angegebenen Bedeutungen haben kann) schließen insbesondere ein:

Verbindungen in denen $R_2$ und $R_3$ bevorzugt zusammen eine Propylen ($-CH_2-CH_2-CH_2-$)- oder eine Butylen ($-CH_2-CH_2-CH_2-CH_2-$)-Gruppe darstellen, in denen gegebenenfalls jeweils eine Methylengruppe ($-CH_2-$) durch -O-, -NH-, oder $-NR_4$-ersetzt sein kann, worin $R_4$ wie vorstehend erwähnt ist, und worin die von $R_2$ und $R_3$ gebildeten Gruppen weiterhin gegebenenfalls jeweils durch einen bis drei Substituenten, ausgewählt aus der Gruppe, die besteht aus Hydroxy, Oxo, $C_{1-4}$-Alkoxy, Amino und Mono- oder Di-($C_{1-4}$-alkyl)amino, substituiert sein können.

Beispielhafte Liganden sind die folgenden:

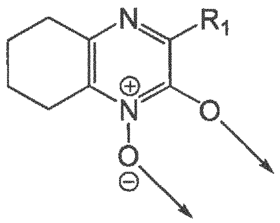 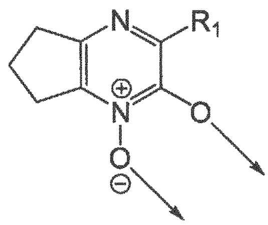

worin $R_1$ jeweils wie vorstehend definiert ist.

Besonders bevorzugt sind insbesondere die Eisen(III)-Komplexverbindungen der allgemeinen Formel (II):

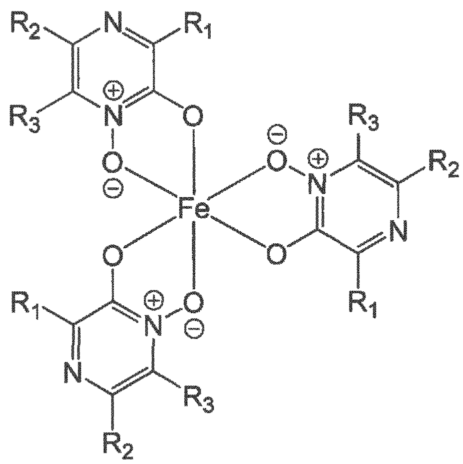

(II)

worin $R_1$, $R_2$, und $R_3$ jeweils wie oben oder bevorzugt wie nachstehend definiert sind.

Bevorzugt sind $R_1$, $R_2$, und $R_3$ gleich oder verschieden und werden ausgewählt aus:

- Wasserstoff,
- $C_{1-6}$-Alkyl, bevorzugt wie vorstehend erläutert,
- Halogen, bevorzugt wie vorstehend erläutert,
- $C_{3-6}$-Cycloalkyl, bevorzugt wie vorstehend erläutert,
- $C_{3-6}$-Cycloalkyl-$C_{1-4}$-alkyl, bevorzugt wie vorstehend erläutert,
- $C_{1-4}$-Alkoxy-$C_{1-4}$-alkyl, bevorzugt wie vorstehend erläutert,
- $C_{1-4}$-Alkoxy-Carbonyl, bevorzugt wie vorstehend erläutert,
- $C_{1-4}$-Mono- oder Dialkylaminocarbonyl, bevorzugt wie vorstehend erläutert,
- Amino-Carbonyl bzw. Carbamoyl ($H_2NCO$-),
- Hydroxy-$C_{1-4}$-alkyl, bevorzugt wie vorstehend erläutert und
- Halogen-$C_{1-4}$-alkyl, bevorzugt wie vorstehend erläutert;

oder

$R_2$ und $R_3$ bilden gemeinsam mit den Kohlenstoffatomen, an die sie gebunden sind, einen gegebenenfalls substituierten 5- oder 6-gliedrigen gesättigten oder ungesättigten Ring, wie vorstehend erläutert.

Besonders bevorzugt sind $R_1$, $R_2$, und $R_3$ gleich oder verschieden und werden ausgewählt aus: Wasserstoff und $C_{1-6}$-Alkyl, bevorzugt wie vorstehend erläutert, insbesondere Wasserstoff, Methyl, Ethyl und Propyl, insbesondere i-Propyl, Butyl, insbesondere sek.-Butyl. Am meisten bevorzugt werden $R_1$, $R_2$, und $R_3$ ausgewählt aus: Wasserstoff, Methyl und Ethyl.

Ebenfalls besonders bevorzugt ist, daß $R_2$ und $R_3$ gemeinsam mit den Kohlenstoffatomen, an die sie gebunden sind, einen gegebenenfalls substituierten 5-oder 6-gliedrigen gesättigten oder ungesättigten Ring, insbesondere einen gegebenenfalls substituierten 5- oder 6-gliedrigen gesättigten Ring, ganz besonders bevorzugt einen 6-gliedrigen gesättigten Ring bilden.

In einer weiteren Ausführungsform der Erfindung betrifft diese die Eisen(III)-pyrazin-2-ol 1-oxid-Komplexverbindungen in fester Form. Der Ausdruck "feste Form" meint hier insbesondere im Unterschied zur gelösten Form, bei

dem die Eisen(III)-pyrazin-2-ol 1-oxid-Komplexverbindungen gelöst in einem Lösungsmittel wie beispielsweise Wasser vorliegen. Der Ausdruck "in fester Form" meint des weiteren, dass die Eisen(III)-pyrazin-2-ol 1-oxid-Komplexverbindungen bei Raumtemperatur (23°C) in fester Form vorliegen. Die Eisen(III)-pyrazin-2-ol 1-oxid-Komplexverbindungen können dabei in amorpher, kristalliner oder teilkristalliner Form vorliegen. Auch können die Eisen(III)-pyrazin-2-ol 1-oxid-Komplexverbindungen der Erfindung als Hydrate vorliegen.

Es ist für den Fachmann klar, dass die erfindungsgemäßen Liganden

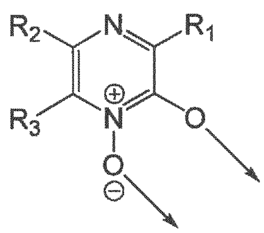

aus den entsprechenden Pyrazin-2-ol-1-oxid-Verbindungen hervorgehen:

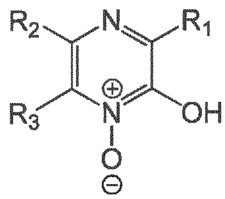

(III)

In den Pyrazin-2-ol-1-oxid-Verbindungen liegt eine Keto-Enol-Tautomerie zwischen 1-Hydroxypyrazin-2(1H) (IIIa) und Pyrazin-2-ol-1-oxid (III) vor, bei der die Gleichgewichtslage durch verschiedene Faktoren bestimmt wird.

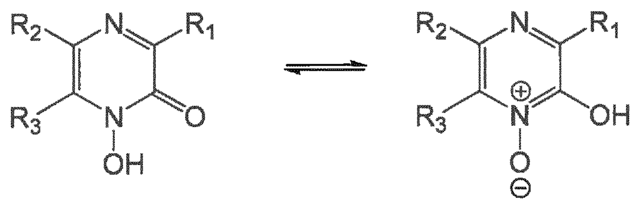

(IIIa) (III)

Der Ligand geht formal durch Abspalten eines Protons aus den entsprechenden Pyrazin-2-ol-1-oxid-Verbindungen (III) hervor:

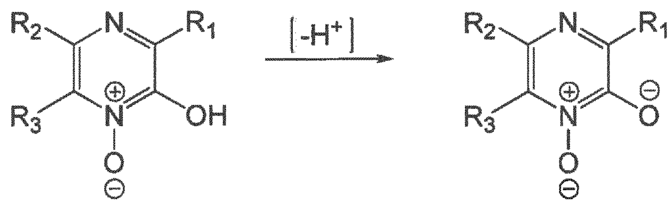


(III)

trägt also formal eine einfach negative Ladung.

Ausserdem ist es für den Fachmann klar, dass die erfindungsgemäßen verwendeten Pyrazin-2-ol-1-oxid-Verbindungen in unterschiedlichen Schreibweisen (a und b) gezeichnet werden können, aber beide den gleichen Sachverhalt des N-Oxids beinhalten.

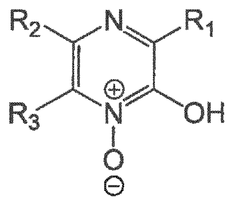

(a)

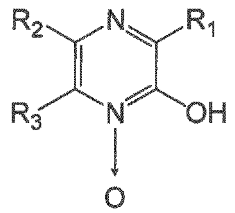

(b)

Die gilt entsprechend auch für die deprotonierte Form der Pyrazin-2-ol-1-oxid-Liganden-Verbindungen. Im Rahmen der Erfindung sind sämtliche tautomere Formen enthalten auch wenn lediglich eine der mesomeren Grenzformeln gezeichnet ist.

Je nach Substituent $R_1$, $R_2$, und $R_3$ können auch diese gegebenenfalls an den tautomeren Grenzstrukturen im Pyrazin-2-ol-1-oxid-Liganden teilhaben, und sämtliche derartige Tautomere sind im Umfang der Erfindung enthalten.

Die erfindungsgemäßen Eisen(III)-pyrazin-2-ol-1-oxid-Komplexverbindungen insbesondere die der allgemeinen Formel (II) bzw. die entsprechenden Pyrazin-2-ol-1-oxid-Liganden können in Form verschiedener Isomere, insbesondere Tautomere vorliegen. Isomere Formen schließen beispielsweise Regioisomere ein, die sich durch die Lage der Liganden zueinander unterscheiden, einschließlich sogenannter optischer Isomere, die sich wie Bild und Spiegelbild zueinander verhalten. Weiterhin können die Liganden bei Vorliegen asymmetrischer Kohlenstoffatome in Form optischer Isomere vorliegen, die sich wie Bild und Spiegelbild zueinander verhalten, und reine Enantiomere, Mischungen der Enantiomere, insbesondere Racemate umfassen. Enantiomerenreine Liganden können wie dem Fachmann bekannt ist durch optische Auflösungsverfahren, wie Umsetzung mit chiralen Reagenzien zu Diastereomeren, Trennung der Diastereomeren und Freisetzen der Enantiomere erhalten werden.

Weitere bevorzugte Ausführungsformen der Erfindung schließen ein:

(In der vorliegenden Erfindung bedeuten die Ziffern 1-6 in "1-6C" oder "C1-6", bzw. "1-4" in "1-4C" oder "C1-4 "etc. jeweils die Anzahl der Kohlenstoffatome der sich daran anschließenden Kohlenwasserstoffreste-Bezeichnungen).

$R_1$, $R_2$ und $R_3$ wird ausgewählt aus der Gruppe, die besteht aus:

- Wasserstoff,
- 1-6C-alkyl, (also Alkyl mit 1 bis 6 Kohlenstoffatomen),
- 3-6C-cycloalkyl,
- 3-6C-cycloalkyl-1-4C-alkyl,
- 1-4C-alkoxy-1-4C-alkyl,
- hydroxy-1-4C-alkyl,

oder $R_2$ und $R_3$ bilden zusammen eine Propylen ($-CH_2-CH_2-CH_2$), Butylen ($-CH_2-CH_2-CH_2-CH_2-$), Azabutylen oder Oxabutylen- Gruppe;
oder $R_2$ und $R_3$ bilden zusammen mit den Kohlenstoffen an die sie gebunden sind, ein ungesättigten Ring, der gegebenenfalls ein oder zwei weitere Heteroatome aufweisen kann,
oder pharmazeutisch verträgliche Salze davon.

Besonders bevorzugt werden die vorstehend genannten Substituentengruppen wie folgt definiert:

**1-6C-Alkyl** beinhaltet bevorzugt geradkettige oder verzweigte Alkylgruppen mit 1 bis 6 Kohlenstoffatomen. Beispiele hierfür können Methyl, Ethyl, n-Propyl, iso-Propyl, n-Butyl, iso-Butyl, sec-Butyl, tert-Butyl n-Pentyl, iso-Pentyl, neo-Pentyl, n-Hexyl, isoHexyl und neo-Hexyl sein.

**3-6C-Cycloalkyl** beinhaltet bevorzugt Cycloalkyl mit 1 bis 6 Kohlenstoffatomen, wie Cyclopropyl, Cyclobutyl, Cyclopentyl, Cyclohexyl.

**3-6C-Cycloalkyl-1-4C-alkyl** beinhaltet bevorzugt eine oben beschriebene 1-6C-Alkyl Gruppe, substituiert mit einer oben beschriebenen 3-6C-Cycloalkyl Gruppe. Beispiele hierfür können eine Cyclopropylmethyl, Cyclopentylmethyl und Cyclohexylmethyl Gruppe sein.

**1-3C-alkoxy-carbonyl-1-6C-alkyl,** beinhaltet bevorzugt eine oben beschriebene 1-6C-Alkyl Gruppe, die mit einer Carbonylgruppe, die mit einer 1-3C Alkoxy Gruppe als Carbonsäureester vorliegt, verknüpft ist. Beispiele hierfür

können ein Methoxycarbonylmethyl, Ethoxycarbonylmethyl, Methoxycarbonylethyl, Ethoxycarbonylethyl und Isopropoxycarbonylmethyl.

**1-4C-Alkoxy** beinhaltet bevorzugt eine 1-4C-Alkoxy Gruppe, bei der ein Sauerstoffatom mit einer geraden oder verzweigten Alkylkette mit 1-4 Kohlenstoffatome verbunden ist. Beispiele dieser Gruppe können Methoxy, Ethoxy, Propoxy und Isobutoxy sein.

**1-4C-Alkoxy-1-4C-alkyl** beinhaltet bevorzugt eine oben beschriebene 1-4C-Alkoxy Gruppe, die mit einer oben beschriebenen 1-4C-alkyl Gruppe verknüpft ist. Beispiele dieser Gruppe können Methoxyethyl, Ethoxypropyl, Methoxypropyl, Isobutoxymethyl sein.

**Hydroxy-1-4C-alkyl** beinhaltet eine oben beschriebene 1-4C-Alkyl Gruppe, die mit einer Hydroxygruppe substituiert ist. Beispiele hier können Hydroxyethyl, Hydroxybutyl und Hydroxyisopropyl sein.

Besonders bevorzugt wird:

**$R_1$ $R_2$** und **$R_3$** ausgewählt aus der Gruppe, die besteht aus:

- Wasserstoff,
- 1-6C-alkyl,
- 1-4C-alkoxy-1-4C-alkyl,
- hydroxy-1-4C-alkyl;

oder **$R_2$** und **$R_3$** bilden zusammen eine Propylen ($-CH_2-CH_2-CH_2$), Butylen ($-CH_2-CH_2-CH_2-CH_2-$), Azabutylen oder Oxabutylen- Gruppe;

oder $R_2$ und $R_3$ bilden zusammen mit den Kohlenstoffen an die sie gebunden sind, ein ungesättigten Ring, der ein weiteres Heteroatom aufweisen kann,

Ganz besonders bevorzugt wird:

**$R_1$, $R_2$** und **$R_3$** ausgewählt aus der Gruppe, die besteht aus:

- Wasserstoff
- 1-6C-alkyl;
- 1-4C-alkoxy-1-4C-alkyl

oder **$R_2$** und **$R_3$** bilden zusammen eine Propylen ($-CH_2-CH_2-CH_2$) oder Butylen ($-CH_2-CH_2-CH_2-CH_2-$) Gruppe;

oder $R_2$ und $R_3$ bilden zusammen mit den Kohlenstoffen an die sie gebunden sind, ein ungesättigten Ring, der ein weiteres Stickstoffatom aufweisen kann.

Besonders bevorzugte Komplex-Verbindungen der allgemeinen Formel (II) sind in den Beispielen beschrieben.
Die Beschreibung betrifft weiterhin ein Verfahren zur Herstellung der erfindungsgemäßen Eisen(III)-Komplexverbindungen, welches die Umsetzung eines Pyrazin-2-ol-1-oxids der Formel (III) mit einem Eisen(III)-salz umfasst.
Pyrazin-2-ol-1-oxide als Ausgangsverbindungen schließen dabei insbesondere diejenigen der Formel (III) ein:

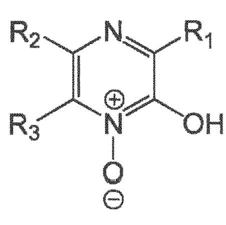

(III)

worin $R_1$, $R_2$ und $R_3$ wie oben definiert sind, wobei auf die tautomeren Grenzstrukturen hingewiesen wurde.
Beispiele geeigneter Eisen(III)-salze schließen ein: Eisen(III)-chlorid, Eisen(III)-acetat, Eisen(III)-sulfat, Eisen(III)-nitrat und Eisen(III)-acetylacetonat, worunter Eisen(III)-chlorid bevorzugt ist.

Ein bevorzugtes Verfahren ist im folgenden Schema gezeigt:

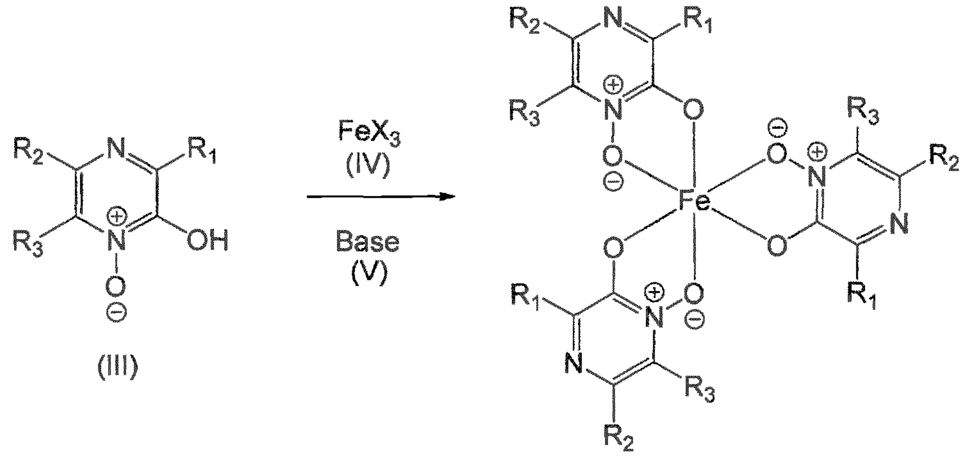


(III)

(II)

worin $R_1$, $R_2$ und $R_3$ wie oben definiert sind, X ein Anion wie Halogenid, wie Chlorid, ein Carboxylat, wie Acetat, Sulfat, Nitrat und Acetylacetonat ist und Base eine übliche organische oder anorganische Base darstellt.

Beim Verfahren werden bevorzugt 3 eq der Pyrazin-2-ol-1-oxide (III) unter Verwendung von geeigneten Eisen(III)-salzen (IV) (besonders eignen sich hier Fe(III)-chlorid, Fe(III)-acetat, Fe(III)-sulfat und Fe(III)-acetylacetonat) zu den entsprechenden Komplexen der allgemeinen Formel (II) unter Standardbedingungen umgesetzt. Hierbei wird die Synthese unter den für die Komplexbildung optimalen pH-Bedingungen durchgeführt. Der optimale pH-Wert wird durch Zugabe von Base (V), besonders eignet sich hier die Verwendung von Natrium-Hydroxyd, NatriumCarbonat, Natrium-Hydrogencarbonat, Natrium-Methanolat, Kalium-Hydroxyd Kaliumcarbonat, Kalium-Hydrogencarbonat oder Kalium-Methanolat, eingestellt.

Die für die Darstellung der Komplexe benötigten Liganden (III) wurden nach folgender Synthesemethode (J. Chem. Soc.1949,2707-2712) hergestellt. Hierzu wurden die käuflichen oder synthetisierten 2-Aminohydroxamsäuren (IV) mit den käuflichen oder synthetisierten 1-2-Dicarbonylverbindungen der allgemeinen Formel (V) unter Standardbedingungen zum Liganden der allgemeinen Formel (III) umgesetzt. Dabei kann es bei Verwendung unsymmetrischer 1-2-Dicarbonylverbindungen zum Auftreten des entsprechenden Regioisomers (IIIa) kommen, welches mit dem Fachmann vertrauten Standardmethoden abgetrennt werden kann. Bei anderen Substitutionsmustern von Diketon (V) kann die Reaktion kann auch weitgehend regioselektiv verlaufen, wie z. B. im Fall von $R_2$ = Methyl, $R_3$ = Wasserstoff.

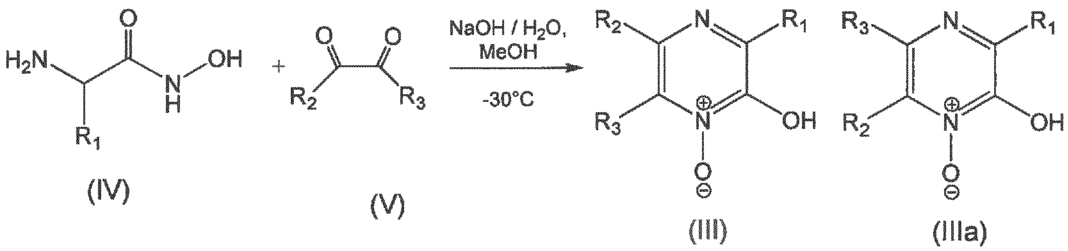


(IV) (V) (III) (IIIa)

Analog hierzu können auch leicht modifizierte Syntheserouten, unter dem Fachmann vertrauten Reaktionsbedingungen, zur Darstellung der entsprechenden Liganden der allgemeinen Formel (III) verwendet werden. So kann nach der Synthese von Ohkanda et.al (Bull. Chem. Soc. Jpn.1993, 66, 841-847) ausgehend von N-terminal geschützen Aminosäuren, der allgemeinen Formel (VI) die entsprechend O-Benzylgeschützten Aminohydroxamsäuren (VII) hergestellt werden, die nach Reaktion mit 1-2-Dicarbonylverbindungen, der allgemeinen Formel (IV) sowie der Abspaltung der O-Benzylgruppe zu Liganden der allgemeinen Formel (III) umgesetzt werden können. Auch bei dieser alternativen Syntheseroute kann es zum Auftreten von (IIIa) kommen.

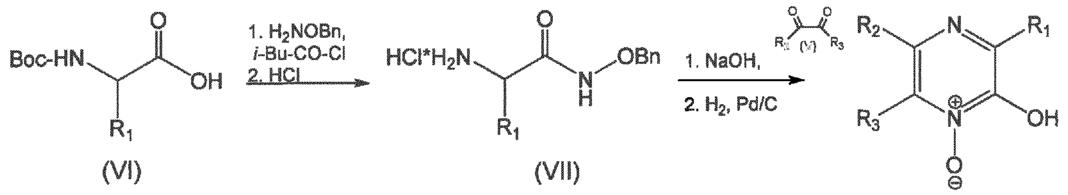

(VI) (VII) (III)

Boc = *t*-Butyloxycarbonyl; Bn = Benzyl

Allgemein lässt sich die Darstellung der Pyrazin-2-ol-1-oxide (III) auch durch andere, dem Fachmann vertraute, Syntheserouten durchführen. Somit besteht beispielsweise die Möglichkeit, die entsprechend substituierten Pyrazine (VIII) durch Umsetzung mit geeigneten Oxidationsmitteln wie Wasserstoffperoxid oder Peroxycarbonsäuren zu den gewünschten Produkten der allgemeinen Formel (III) darzustellen (z. B. J. Org. Chem.1958, 23, 1603-1606), wobei sowohl die Regiochemie der Oxidation als auch der Oxidationsgrad (einfach oder doppelt) von den Reaktionsbedingungen, insbesondere dem Oxidationsreagenz und dem Substitutionsmuster des entsprechenden Pyrazins bestimmt werden (z. B. J. Heterocycl. Chem.1983, 19, 1061, J. Heterocycl. Chem.1989, 26, 812).

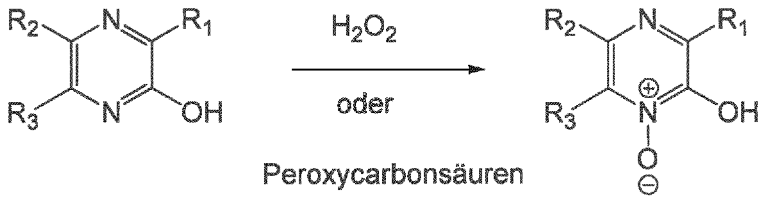

(VIII) (III)

Beispiele der Pyrazin-2-ol 1-oxid-Ausgangsverbindungen (III) schließen insbesondere die folgenden ein:

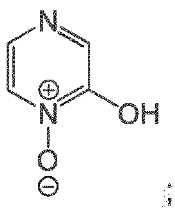 ; 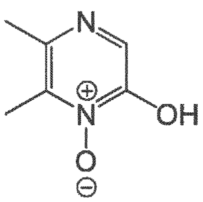 ; 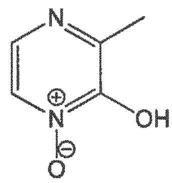 ; 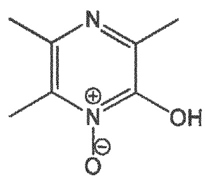 ; 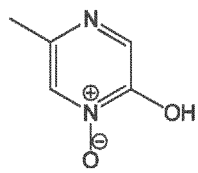 ;

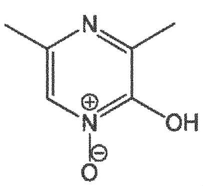 ; 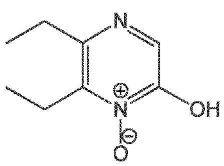 ; 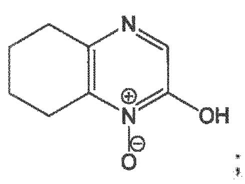 ; 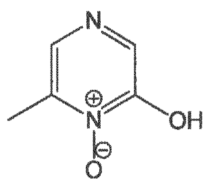 ; 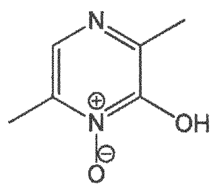 .

Aus diesen Verbindungen gehen die Liganden der erfindungsgemäßen Eisen-Komplexverbindungen durch einfache Deprotonierung an der Hydroxygruppe hervor.

Pharmazeutisch annehmbare Salze der erfindungsgemäßen Verbindungen, in denen der Eisen(III)-Komplex formal eine positive Ladung trägt, schließen beispielsweise Salze mit geeigneten Anionen ein, wie Carboxylate, Sulfonate, Sulfate, Chloride, Bromide, Iodide, Phosphate, Tartrate, Methansulfonate, Hydroxyethansulfonate, Glycinate, Maleate, Propionate, Fumarate, Toluolsulfonate, Benzolsulfonate,Trifluoracetate, Naphthalindisulfonate-1,5, Salicylate, Benzoate, Lactate, Salze der Äpfelsäure, Salze der 3-Hydroxy-2-naphthoesäure-2, Citrate und Acetate ein.

Pharmazeutisch annehmbaren Salze der erfindungsgemäßen Verbindungen, in denen der Eisen(III)-Komplex formal eine negative Ladung trägt, schließen beispielsweise Salze mit geeigneten pharmazeutisch annehmbaren Basen ein, wie z.B. Salze mit Alkali- oder Erdalkalihydroxiden, wie NaOH, KOH, $Ca(OH)_2$, $Mg(OH)_2$ etc., Aminverbindungen, wie Ethylamin, Diethylamin, Triethylamin, Ethyldiisopropylamin, Ethanolamin, Diethanolamin, Triethanolamin, Methylglucamin, Dicyclohexylamin, Dimethylaminoethanol, Prokain, Dibenzylamin, N-Methylmorpholin, Arginin, Lysin, Ethylendiamin, N-Methylpiperidin 2-Amino-2-methyl-propanol-(1), 2-Amino-2-methyl-propandiol-(1,3), 2-Amino-2-hydroxylmethyl-propandiol-(1,3) (TRIS) etc..

Die Wasserlöslichkeit bzw. die Löslichkeit in physiologischer Kochsalzlösung und damit gegebenenfalls auch die Wirksamkeit der erfindungsgemäßen Verbindungen kann durch Salzbildung generell, speziell auch durch die Wahl des Gegenions signifikant beeinflusst werden.

Bevorzugt stellen die erfindungsgemäßen Verbindungen neutrale Komplex-Verbindungen dar.

**Vorteilhafte pharmakologische Effekte:**

Die Erfinder fanden überraschend, dass die Eisen(III)-pyrazin-2-ol 1-oxid-Komplexverbindungen, die Gegenstand der vorliegenden Erfindung sind, und die insbesondere durch die allgemeine Strukturformel (II) dargestellt werden, stabile bioverfügbare Eisenkomplexe sind und zur Verwendung als Arzneimittel zur Behandlung und Prophylaxe von Eisenmangelerscheinungen und Eisenmangelanämien sowie der damit einhergehenden Symptome geeignet sind.

Die erfindungsgemäßen Verbindungen enthaltende Arzneimittel eignen sich zum Einsatz in der Human- und der Veterinärmedizin.

Die erfindungsgemäßen Verbindungen eignen sich somit auch zur Herstellung eines Medikamentes zur Behandlung von Patienten, die unter Symptomen einer Eisenmangelanämie wie zum Beispiel: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit oder depressiven Verstimmungen leiden.

Die erfindungsgemäßen Eisen(III)-Komplexverbindungen eignen sich weiterhin zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF; congestive heart failure), Eisenmangelanämien bei chronischer Niereninsuffizienz Stadium 3 -5 (CDK 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases).

Die Verabreichung kann über einen Zeitraum von mehreren Monaten bis zur Verbesserung des Eisenstatus, reflektiert beispielsweise durch den Hämoglobin-Wert, die Transferrin-Sättigung und den Serum Ferritin-Wert der Patienten, oder zur gewünschten Verbesserung einer durch Eisenmangelanämie hervorgerufenen Beeinträchtigung des Gesundheitszustandes erfolgen.

Das erfindungsgemäße Präparat kann von Kindern, Jugendlichen und Erwachsenen eingenommen werden.

Die erfindungsgemäß angewendeten Verbindungen können dabei sowohl oral als auch parenteral verabreicht werden. Bevorzugt ist die orale Verabreichung.

Die erfindungsgemäßen Verbindungen sowie die vorgenannten Kombinationen der erfindungsgemäßen Verbindungen mit weiteren Wirkstoffen oder Arzneimitteln können somit insbesondere zur Herstellung von Medikamenten zur Behandlung der Eisenmangelanämie verwendet werden, wie Eisenmangelanämien bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), Menstruation, Verletzungen, Eisenmangelanämie infolge von Psilosis (sprue), Eisenmangelanämie infolge verminderter Eisenaufnahme bei der Ernährung, insbesondere bei selektiv essenden Kindern und Heranwachsenden, Immunschwäche hervorgerufen durch Eisenmangelanämie, Beeinträchtigung der Hirnleistung hervorgerufen durch Eisenmangelanämie, Restless Leg Syndrom.

Die erfindungsgemäße Anwendung führt zu einer Verbesserung der Eisen-, Hämoglobin-, Ferritin- und Transferrinwerte, welche insbesondere bei Heranwachsenden und Kindern aber auch bei Erwachsenen mit einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion), oder zu einer Verbesserung der Neutrophilen-Niveaus, der Antikörperniveaus und/oder der Lymphozytenfunktion einhergeht.

Die vorliegende Erfindung betrifft weiterhin pharmazeutische Zusammensetzungen, enthaltend eine oder mehrere der erfindungsgemäßen Verbindungen, insbesondere nach Formel (II), sowie gegebenenfalls eine oder mehrere weitere pharmazeutisch wirksame Verbindungen sowie gegebenenfalls einen oder mehrere pharmakologisch verträgliche Träger und/oder Hilfsstoffe und/oder Lösungsmittel. Die genannten pharmazeutische Zusammensetzungen enthalten beispielsweise bis 99 Gew.-% oder bis zu 90 Gew.-% oder bis zu 80 Gew.-% oder bis zu 70 Gew.-% der erfindungsgemäßen Verbindungen, wobei der Rest jeweils durch pharmakologisch verträgliche Träger und/oder Hilfsstoffe und/oder Lösungsmittel gebildet wird.

Dabei handelt es sich um übliche pharmazeutische Träger, Hilfsstoffe oder Lösungsmittel. Genannte pharmazeutische Zusammensetzungen sind beispielsweise geeignet zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistenten Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Granulaten, Suppositorien, Emulsionen, Dispersionen, Mikrokapseln, Mikroformullerungen, Nanoformulierungen, liposomalen Formulierungen, Kapseln, magensaftresistenten Kapseln, Pulvern, Inhalationspulvern, mikrokristallinen Formulierungen, Inhalationssprays, Puder, Tropfen, Nasentropfen, Nasensprays, Aerosolen, Ampullen, Lösungen, Säften, Suspensionen, Infusionslösungen oder Injektionslösungen etc. vor.

Bevorzugt werden die erfindungsgemäßen Verbindungen sowie pharmazeutische Zusammensetzungen, enthaltend solche Verbindungen oral appliziert, obwohl auch andere Formen wie parenteral, insbesondere intravenös möglich sind.

Dazu liegen die erfindungsgemäßen Verbindungen bevorzugt in pharmazeutischen Zusammensetzungen in Form von Pillen, Tabletten, magensaftresistenten Tabletten, Filmtabletten, Schichttabletten, Retardformulierungen zur oralen Verabreichung, Depotformulierungen, Dragees, Granulaten, Emulsionen, Dispersionen, Mikrokapseln, Mikroformulierungen, Nanoformulierungen, liposomalen Formulierungen, Kapseln, magensaftresistenten Kapseln, Pulvern, mikrokristallinen Formulierungen, Puder, Tropfen, Ampullen, Lösungen, Suspensionen, Infusionslösungen oder Injektionslösungen vor.

In einer bevorzugten Ausführungsform der Erfindung werden die Eisen-Komplexverbindungen in Form einer Tablette oder Kapsel verabreicht. Diese können beispielsweise als säureresistente Formen oder mit pH-abhängigen Beschichtungen vorliegen.

Die erfindungsgemäßen Verbindungen können in pharmazeutischen Zusammensetzungen verabreicht werden, die verschiedene organische oder anorganische Träger- und/oder Hilfsmaterialien enthalten können, wie sie üblicherweise für pharmazeutische Zwecke insbesondere für feste Arzneimittelformulierungen verwendet werden, wie beispielsweise Exzipienten (wie Saccharose, Stärke, Mannit, Sorbit, Lactose, Glucose, Cellulose, Talk, Calciumphosphat, Calciumcarbonat), Bindemittel (wie Cellulose, Methylcellulose, Hydroxypropylcellulose, Polypropylpyrrolidon, Gelatine, Gummiarabicum, Polyethylenglykol, Saccharose, Stärke), Desintegrationsmittel (wie Stärke, hydrolysierte Stärke, Carboxymethylcellulose, Calciumsalz von Carboxymethylcellulose, Hydroxypropylstärke, Natriumglycolstärke, Natriumbicarbonat, Calciumphosphat, Calciumcitrat), Gleit- bzw. Schmiermittel (wie Magnesiumstearat, Talk, Natriumlaurylsulfat), ein Geschmacksbildner (wie Citronensäure, Menthol, Glycin, Orangenpulver), Konservierungsmittel (wie Natriumbenzoat, Natriumbisulfit, Methylparaben, Propylparaben), Stabilisatoren (wie Citronensäure, Natriumcitrat, Essigsäure, und Multicarbonsäuren aus der Titriplex Reihe wie z.B. Diethylentriaminpentaessigsäure (DTPA), Suspendiermittel (wie Methylcellulose, Polyvinylpyrrolidon, Aluminiumstearat), Dispergiermittel, Verdünnungsmittel (wie Wasser, organische Lösungsmittel), Bienenwachs, Kakaobutter, Polyethylenglykol, weißes Petrolatum etc..

Flüssige Arzneimittelformulierungen, wie Lösungen, Suspensionen und Gele enthalten üblicherweise einen flüssigen Träger, wie Wasser und/oder pharmazeutisch verträgliche organische Lösungsmittel. Weiterhin können derartige flüssige Formulierungen auch pH-einstellende Mittel, Emulgatoren oder dispergierende Agenzien, puffernde Agenzien, Konservierungsmittel, Netzmittel, Geliermittel (beispielsweise Methylcellulose), Färbemittel und/oder Aromastoffe enthalten. Die Zusammensetzungen können isotonisch sein, das heisst diese können den gleichen osmotischen Druck wie Blut haben. Die Isotonie der Zusammensetzung kann durch die Verwendung von Natriumchlorid oder anderer pharmazeutisch annehmbare Agenzien wie beispielsweise Dextrose, Maltose, Borsäure, Natriumtartrat, Propylenglykol oder andere anorganische oder organisch lösliche Substanzen eingestellt werden. Die Viskosität der flüssigen Zusammensetzungen kann unter Verwendung eines pharmazeutisch annehmbaren Verdickungsmittels, wie Methylcellulose eingestellt werden. Andere geeignete Verdickungsmittel umfassen beispielsweise Xanthan, Carboxymethylcellulose, Hydroxypropylcellulose, Carbomer und dergleichen. Die bevorzugte Konzentration des Verdickungsmittels wird von dem ausgewählten Agens abhängen. Pharmazeutisch annehmbare Konservierungsmittel können verwendet werden, um die Haltbarkeit der flüssigen Zusammensetzung zu erhöhen. Benzylalkohol kann geeignet sein, obwohl eine Vielzahl von Konservierungsmitteln einschließlich beispielsweise Paraben, Thimerosal, Chlorbutanol oder Benzalkoniumchlorid ebenfalls verwendet werden können.

Der Wirkstoff kann beispielsweise mit einer Einheitsdosis von 0,001 mg/kg bis 500 mg/kg Körpergewicht beispielweise bis zu 1 bis 4 mal am Tag verabreicht werden. Die Dosierung kann jedoch je nach Alter, Gewicht, Zustand des Patienten, Schwere der Erkrankung oder Art der Verabreichung erhöht oder herabgesetzt werden.

## EXPERIMENTELLER TEIL

Die Ligandbezeichnungen wurden nach IUPAC-Nomenklatur mit dem Programm ACD/Name, Version 12.01 von Advanced Chemistry Development Inc. erstellt.

Abkürzungen

| | | | |
|---|---|---|---|
| s | Singulett | t | Triplett |
| d | Duplett | q | Quartett |
| dd | Doppel-Duplett | m | Multiplett (breit/überlagert) |
| L | Ligand | | |

## Ausgangsverbindungen:

**A. Pyrazin-2-ol 1-oxid**

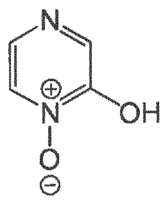

Die Synthese wurde analog folgender Literatur durchgeführt: G. Dunn et al., J. Chem. Soc. 1949, 2707-2712.

B. 5,6-Dimethyl-pyrazin-2-ol **1-oxid**

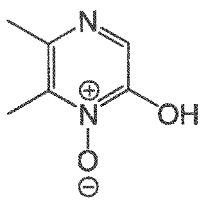

Die Synthese wurde analog folgender Literatur: G. Dunn et al., J. Chem. Soc.1949,2707-2712 durchgeführt.

C. **3-Methylpyrazin-2-ol-1-oxid**

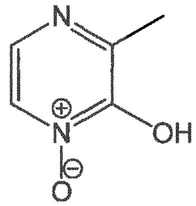

Die Synthese wurde analog folgender Literatur durchgeführt: G. Dunn et al., J. Chem. Soc. 1949, 2707-2712.

D. **3,5,6-Trimethylpyrazin-2-ol-1-oxid**

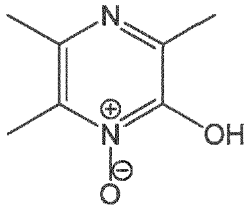

Die Synthese wurde analog folgender Literatur durchgeführt: G. Dunn et al., J. Chem. Soc. 1949, 2707-2712.

**E. 5-Methylpyrazin-2-ol-1-oxid**

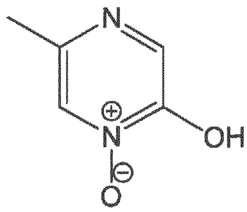

100 mmol (9 g) Glycin-hydroxamsäure wurden in je 200 ml Wasser und Methanol auf -25°C gekühlt, 100 mmol (15.4 ml 40% Lösung) Methylglyoxal zugetropft und anschließend mit 10 ml 30% NaOH ca. pH 11 eingestellt. In 2 h wurde auf 5°C erwärmt und unter Vakuum auf die Hälfte des Lösungsmittelvolumens eingeengt. Mit 20% HCl wurde auf pH 3 eingestellt, ausgefallener Feststoff abfiltriert und getrocknet. Man erhielt 7.28 g (52% Ausbeute) der Titelverbindung.

IR (in Substanz, $cm^{-1}$): 1644, 1577, 1527, 1454, 1425, 1381, 1333, 1229, 1141, 1062, 1028, 949, 896, 835, 806, 732.
CHN-Elementaranalyse: C, 42.91; H, 4.49; N, 19.54.
1 H-NMR (DMSO-$d_6$, 400 MHz): $\delta$ [ppm] = 8.04 (s, 1 H), 7.83 (s, 1 H), 2.21 (s, 3H).

**F. 3,5-Dimethylpyrazin-2-ol-1-oxid**

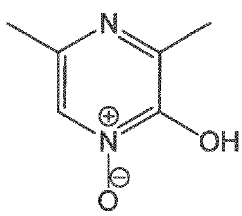

407 mmol (42.4 g) Alanin-hydroxamsäure wurden in je 200 ml Wasser und Methanol auf -10°C gekühlt, 400 mmol (57 ml 40% Lösung) Methylglyoxal zugetropft und anschließend mit 19 ml 30% NaOH ca. pH 11 eingestellt. Innerhalb von 2 h wurde auf 0°C erwärmt und dann mit 20% HCl auf pH 3 eingestellt. Die Lösung wurde mit Vakuumdestillation zur Trockne eingeengt, der Rückstand mit 50 ml Ethanol auf Rückfluss erwärmt, nach dem Abkühlen filtriert und das Filtrat erneut zur Trockne eingeengt. 44 g des festen Rückstands wurden in 50 ml Wasser gelöst und mit 15 mmol (4.06 g) $FeCl_3*6H_2O$ versetzt, 30 min auf 50°C geheizt, eingeengt und 4 h bei 5°C stehen gelassen. Der ausgefallene Feststoff wurde abfiltriert und getrocknet. 3.7 g Feststoff wurden in 50 ml Wasser und 10 ml Ethanol mit 6 ml 1 M NaOH auf pH 11 eingestellt, über Nacht gerührt und abzentrifugiert. Der Überstand wurde abdekantiert, mit 3 ml 1 M HCl auf pH 3 eingestellt und zur Trockne eingeengt. 4.2 g Feststoff wurden in 20 ml Ethanol rückflussiert, abgekühlt und abfiltriert. Das Filtrat wurde erneut zur Trockne eingeengt und die erhaltenen 2.8 g Feststoff mit 10 ml Wasser aufgeschlämmt, auf 50°C geheizt und über Nacht abkühlen gelassen. Die Suspension wurde zentrifugiert, der Überstand abdekantiert und zur Trockne eingeengt. Man erhielt 2.0 g (3.5% Ausbeute) der Titelverbindung.

IR (in Substanz, $cm^{-1}$): 1633, 1579, 1526, 1423, 1372, 1318, 1274, 1212, 1148, 1030, 987, 934, 829, 751, 681, 613.
LC-MS (m/z): 141.6 (M+H).
CHN-Elementaranalyse: C, 50.15; H, 5.75; N, 18.83.
1 H-NMR (DMSO-$d_6$, 400 MHz): $\delta$ [ppm] = 7.64 (s, 1 H), 2.31 (s, 3H), 2.15 (s, 3H).

**G. 5,6-Diethylpyrazin-2-ol-1-oxid**

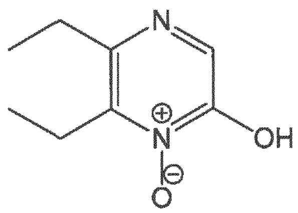

Die Synthese wurde analog folgender Literatur durchgeführt: G. Dunn et al., J. Chem. Soc. 1949, 2707-2712.

**H. 5,6,7,8-Tetrahydroquinoxalin-2-ol-1-oxid**

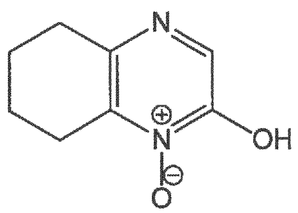

Die Synthese wurde analog folgender Literatur durchgeführt: G. Dunn et al., J. Chem. Soc. 1949, 2707-2712.

I. **6-Methylpyrazin-2-ol-1-oxid**

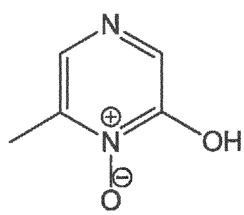

156 mmol (20 g) 2-Chlor-6-methylpyrazin wurden in 80 ml 96% Schwefelsäure gelöst, 222 mmol (63 g) Kaliumperoxodisulfat portionsweise bei 10°C zugegeben und 2 Tage bei 10°C sowie 1 Tag bei Raumtemperatur gerührt (vgl. C.E. Mixan, R. Garth, J. Org. Chem. 1977, 42, 1869-1871). Die Reaktionsmischung wurde auf 200 g Eis gegossen, unter Zugabe von Ethanol mit Calciumhydroxid neutralisiert, dann abfiltriert und mit Ethanol nachgewaschen. Das Filtrat wurde zur Trockne eingedampft, der erhaltene Feststoff (20 g) wurde 3 h in 300 ml 20% KOH Lösung rückflussiert und nach Abkühlen mit 20% HCl auf pH 3 eingestellt. Die Lösung wurde zur Trockne eingedampft, der Rückstand 2 h mit 0.2 l Ethanol am Rückfluss gekocht, nach dem Abkühlen filtriert und das Filtrat zur Trockne eingedampft. Aus 7.4 g Rohprodukt wurden nach Umkristallisation aus Ethanol/Tetrahydrofuran 2.17 g (10% Ausbeute, 93% Reinheit) der Titelverbindung erhalten.

IR (in Substanz, $cm^{-1}$): 1649, 1573, 1541, 1454, 1413, 1389, 1374, 1297, 1247, 1208, 1171, 1126, 1055, 1037, 981, 896, 848, 816, 718.
LC-MS (m/z): 127.4 (M+H).
CHN-Elementaranalyse: C, 44.77; H, 4.56; N, 20.72.
1 H-NMR (DMSO-$d_6$, 400 MHz): $\delta$ [ppm] = 7.95 (s, 1 H), 7.30 (s, 1 H), 2.31 (s, 3H).

**J. 3,6-Dimethylpyrazin-2-ol-1-oxid**

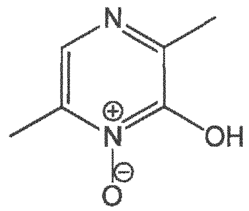

Die Synthese wurde analog folgender Literatur durchgeführt: A. Ohta et al., J. Heterocyclic Chem. 1981, 18, 555-558.

**Eisen-Komplexverbindungen (Beispiele)**

**Beispiel 1**

**Tris-(pyrazin-2-ol 1-oxid)-eisen(III)-Komplex**

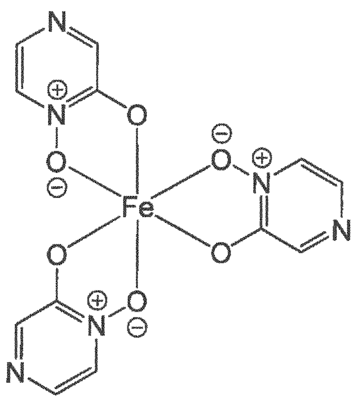

15 mmol (1.77 g) Pyrazin-2-ol **1-oxid** (analog G. Dunn et al., J. Chem. Soc.1949,2707-2712) wurden in 50 ml Ethanol gelöst, auf Rückfluss erhitzt und 5 mmol (1.352 g) in 20 ml Ethanol gelöstes $FeCl_3*6H_2O$ zugegeben. Unter Rückfluss wurden 50 ml Wasser zugetropft und die Reaktionsmischung abkühlen gelassen. Anschliessend wurde mit 15 ml 1 M NaOH auf pH 4.5 eingestellt, 2 h bei 50°C gerührt und nach dem Abkühlen das Produkt abfiltriert und bei 50°C im Vakuumtrockenschrank getrocknet. Man erhielt 1.89g (95% Fe-Ausbeute) der Titelverbindung.

IR (in Substanz, $cm^{-1}$): 3096, 3060, 1595, 1517, 1476, 1440, 1340, 1289, 1188, 1157, 1048, 921, 890, 830, 801.

Fe-Gehalt: 13.8% [m/m]
Chlorid-Gehalt: 3.0% [m/m]

**Beispiel 2**

**Tris-(5,6-dimethylpyrazin-2-ol 1-oxid)-eisen(III)-Komplex**

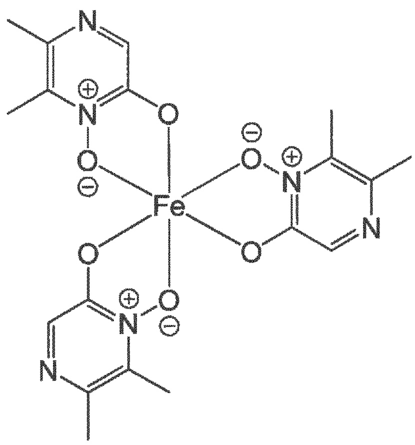

15 mmol (2.213 g) 5,6-Dimethylpyrazin-2-ol 1-oxid(analog G. Dunn et al., J. Chem. Soc.1949,2707-2712) wurden in 50 ml Ethanol gelöst, auf Rückfluss erhitzt und 5 mmol (1.352 g) in 25 ml Ethanol gelöstes $FeCl_3*6H_2O$ zugegeben. Unter Rückfluss wurden 75 ml Wasser zugetropft und die Reaktionsmischung abkühlen gelassen. Anschließend wurde mit 10 ml 1 M NaOH auf pH 4.5 eingestellt, 2 h bei 50°C gerührt und nach dem Abkühlen das Produkt abfiltriert und bei 50°C im Vakuumtrockenschrank getrocknet. Man erhielt 2.07g (86% Fe-Ausbeute) der Titelverbindung.

IR (in Substanz, $cm^{-1}$): 3037, 1594, 1477, 1361, 1276, 1217, 1161, 1093, 1065, 1018, 968, 878, 771, 692.
Fe-Gehalt: 11.63% [m/m]
Chlorid-Gehalt: 0.0% [m/m] (nicht nachweisbar)

**Beispiel 3**

**Tris-(3-Methylpyrazin-2-ol-1-oxid)-eisen(III)-Komplex**

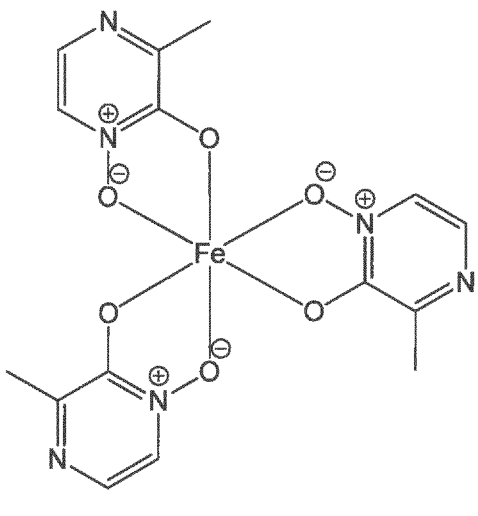

12 mmol (1.51 g) 3-Methylpyrazin-2-ol-1-oxid (analog G. Dunn et al., J. Chem. Soc. 1949, 2707-2712) wurden

in 100 ml Ethanol auf Rückfluss erhitzt und 4 mmol (1.08 g) in 20 ml Ethanol gelöstes $FeCl_3*6H_2O$ zugegeben. Unter Rückfluss wurden 120 ml Wasser zugetropft, die Hälfte der Lösungsmittel abdestilliert und die Reaktionsmischung abkühlen gelassen. Anschließend wurde mit 9 ml 1 M NaOH auf pH 4.4 eingestellt und noch 15 min bei 50°C gerührt. Nach dem Abkühlen wurde das Produkt abfiltriert und getrocknet. Man erhielt 1.57 g (89% Fe-Ausbeute) der Titelverbindung.

IR (in Substanz, $cm^{-1}$): 1588, 1523, 1487, 1373, 1333, 1314, 1257, 1185, 1099, 1032, 946, 881, 809, 793, 752, 729.
CHN-Elementaranalyse: C, 41.92; H, 3.58; N, 19.33.
Fe-Gehalt: 12.7% [m/m]

Chlorid-Gehalt: 0.0% [m/m] (nicht nachweisbar)

**Beispiel 4**

**Tris-(3,5,6-trimethylpyrazin-2-ol-1-oxid)-eisen(III)-Komplex**

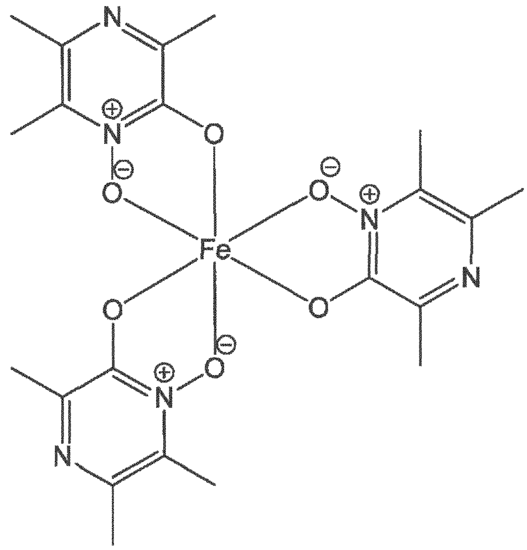

39 mmol (6.0 g) 3,5,6-Trimethylpyrazin-2-ol-1-oxid (analog G. Dunn et al., J. Chem. Soc. 1949, 2707-2712) wurden in 80 ml Ethanol auf Rückfluss erhitzt und 13 mmol (3.5 g) in 10 ml Ethanol gelöstes $FeCl_3*6H_2O$ zugegeben. Unter Rückfluss wurden 80 ml Wasser zugetropft, die Hälfte der Lösungsmittel abdestilliert und die Reaktionsmischung abkühlen gelassen. Anschließend wurde mit 48 ml 1 M NaOH auf pH 4.4 eingestellt und noch 15 min bei 50°C gerührt. Nach dem Abkühlen wurde das Produkt abfiltriert und getrocknet. Man erhielt 6.48 g (90% Fe-Ausbeute) der Titelverbindung.

IR (in Substanz, $cm^{-1}$): 1587, 1486, 1413, 1363, 1332, 1182, 1129, 1082, 1005, 940, 873, 749, 721.
CHN-Elementaranalyse: C, 47.67; H, 5.26; N, 15.82.
Fe-Gehalt: 10.07% [m/m]
Chlorid-Gehalt: 0.0% [m/m] (nicht nachweisbar)

**Beispiel 5**

**Tris-(5-methylpyrazin-2-ol-1-oxid)-eisen(III)-Komplex**

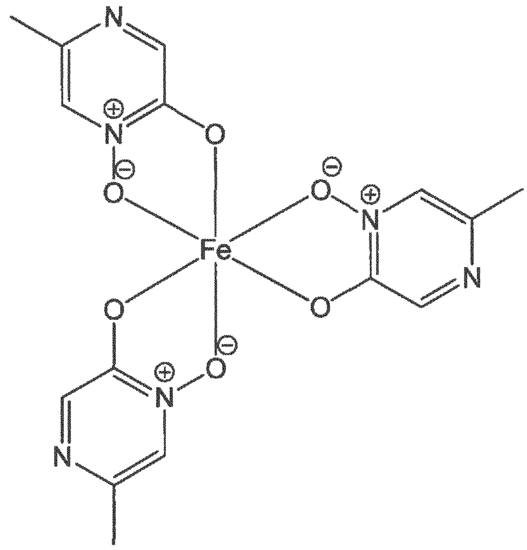

42.8 mmol (6.0 g, 90% Reinheit) 5-Methylpyrazin-2-ol-1-oxid wurden in 360 ml Ethanol auf Rückfluss erhitzt und 14.3 mmol (3.86 g) in 20 ml Ethanol gelöstes $FeCl_3*6H_2O$ zugegeben. Unter Rückfluss wurden 380 ml Wasser zugetropft, die Hälfte der Lösungsmittel abdestilliert und die Reaktionsmischung abkühlen gelassen. Anschließend wurde mit 45 ml 1 M NaOH auf pH 4.4 eingestellt und noch 30 min bei 50°C gerührt. Nach dem Abkühlen wurde das Produkt abfiltriert und getrocknet. Man erhielt 5.31 g (80% Fe-Ausbeute) der Titelverbindung.

IR (in Substanz, $cm^{-1}$): 1611, 1514, 1486, 1450, 1398, 1380, 1340, 1256, 1205, 1184, 1122, 1039, 1007, 939, 918, 873, 840, 757, 742.
CHN-Elementaranalyse: C, 40.88; H, 3.55; N, 18.86.
Fe-Gehalt: 11.96% [m/m]
Chlorid-Gehalt: 0% [m/m] (nicht nachweisbar)

**Beispiel 6**

**Tris-(3,5-dimethylpyrazin-2-ol-1-oxid)-eisen(III)-Komplex**

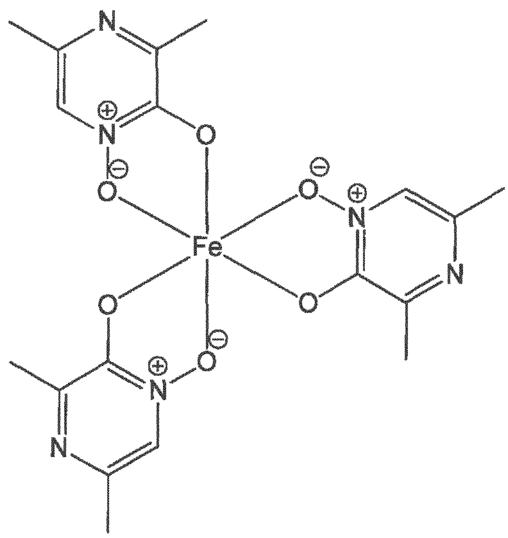

11.4 mmol (1.68 g) 3,5-Dimethylpyrazin-2-ol-1-oxid wurden in 50 ml Ethanol auf Rückfluss erhitzt und 3.8 mmol (1.03 g) in 10 ml Ethanol gelöstes $FeCl_3*6H_2O$ zugegeben. Unter Rückfluss wurden 50 ml Wasser zugetropft, die Hälfte der Lösungsmittel abdestilliert und die Reaktionsmischung abkühlen gelassen. Anschließend wurde mit 7 ml 1 M NaOH auf pH 4.4 eingestellt und nach Abdestillieren weiterer 30 ml Lösungsmittel über Nacht gerührt. Das Produkt wurde

abfiltriert und getrocknet, man erhielt 1.11 g (47% Fe-Ausbeute) der Titelverbindung.

IR (in Substanz, $cm^{-1}$): 1526, 1489, 1373, 1332, 1306, 1226, 1141, 1055, 940, 834, 748, 643, 605.
Fe-Gehalt: 9.07% [m/m]
Chlorid-Gehalt: 0.79% [m/m]

**Beispiel 7**

**Tris-(5,6-diethylpyrazin-2-ol-1-oxid)-eisen(III)-Komplex**

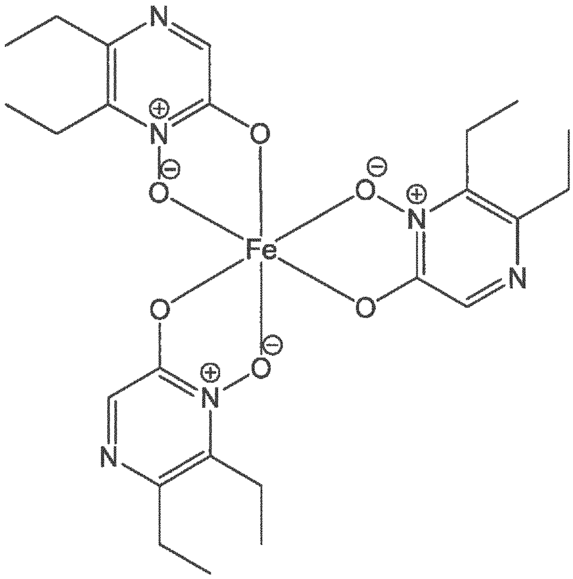

6.6 mmol (1.1 g) 5,6-Diethylpyrazin-2-ol-1-oxid wurden in 30 ml Ethanol auf Rückfluss erhitzt und 2.2 mmol (0.6 g) in 10 ml Ethanol gelöstes $FeCl_3*6H_2O$ zugegeben. Unter Rückfluss wurden 35 ml Wasser zugetropft, die Hälfte der Lösungsmittel abdestilliert und die Reaktionsmischung abkühlen gelassen. Anschließend wurde mit 3.8 ml 1 M NaOH auf pH 4.2 eingestellt, das Produkt abfiltriert und getrocknet. Man erhielt 1.15 g (99% Fe-Ausbeute) der Titelverbindung.

IR (in Substanz, $cm^{-1}$): 2974, 2937, 2876, 1706, 1588, 1509, 1479, 1457, 1353, 1260, 1214, 1158, 1105, 1048, 981, 950, 895, 782, 754, 687, 667, 608.
CHN-Elementaranalyse: C, 51.5; H, 6.27; N, 14.31.
Fe-Gehalt: 10.6% [m/m]
Chlorid-Gehalt: 3.4% [m/m]

**Beispiel 8**

**Tris-(5,6,7,8-tetrahydroquinoxalin-2-ol-1-oxid)-eisen(III)-Komplex**

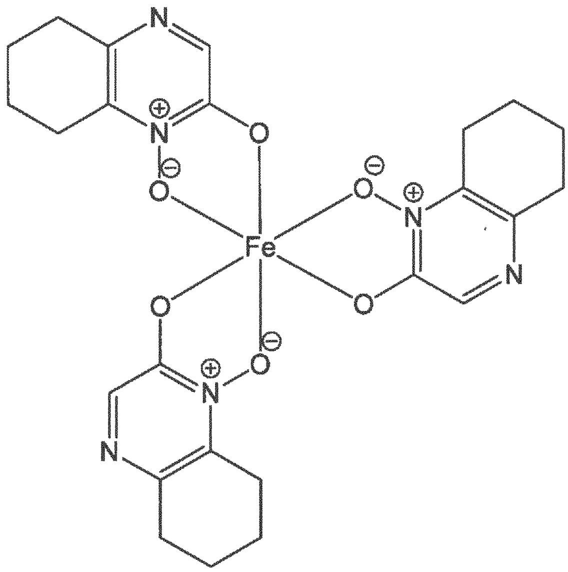

15 mmol (2.62 g) 5,6,7,8-Tetrahydroquinoxalin-2-ol-1-oxid wurden in 100 ml Ethanol auf Rückfluss erhitzt und 5 mmol (1.35 g) in 30 ml Ethanol gelöstes $FeCl_3$*$6H_2O$ zugegeben. Unter Rückfluss wurden 100 ml Wasser zugetropft, die Hälfte der Lösungsmittel abdestilliert und die Reaktionsmischung abkühlen gelassen. Anschließend wurde mit 15 ml 1 M NaOH auf pH 4.5 eingestellt, das Produkt abfiltriert und getrocknet. Man erhielt 2.52 g (91% Fe-Ausbeute) der Titelverbindung.

IR (in Substanz, $cm^{-1}$): 2933, 1592, 1519, 1486, 1433, 1366, 1325, 1266, 1245, 1217, 1184, 1134, 1102, 1067, 987, 966, 887, 856, 821, 762, 690, 633, 611.
CHN-Elementaranalyse: C, 52.44; H, 5.03; N, 15.21.
Fe-Gehalt: 10.12% [m/m] Chlorid-Gehalt: 0.0% [m/m] (nicht nachweisbar)

**Beispiel 9**

**Tris-(6-methylpyrazin-2-ol-1-oxid)-eisen(III)-Komplex**

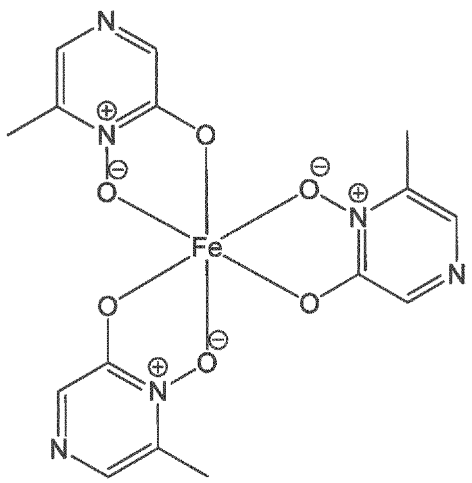

15.4 mmol (1.94 g) 6-Methylpyrazin-2-ol-1-oxid wurden in 30 ml Ethanol auf Rückfluss erhitzt und 5.1 mmol (1.39 g) in 5 ml Ethanol gelöstes $FeCl_3$*$6H_2O$ zugegeben. Unter Rückfluss wurden weitere 45 ml Ethanol und 75 ml Wasser zugetropft, 120 ml Lösungsmittel abdestilliert und die Reaktionsmischung abkühlen gelassen. Anschließend wurde mit 15 ml 1 M NaOH auf pH 4.4 eingestellt, 30 min bei 50°C nachgerührt, dann das Produkt abfiltriert und getrocknet. Man erhielt 2.1g (92% Fe-Ausbeute) der Titelverbindung.

IR (in Substanz, $cm^{-1}$): 1597, 1528, 1478, 1409, 1379, 1357, 1218, 1189, 1170, 1134, 1080, 1035, 989, 924, 861, 828, 712.
CHN-Elementaranalyse: C, 40.68 ; H, 3.48; N, 18.77.
Fe-Gehalt: 12.62% [m/m]
Chlorid-Gehalt: 0.0% [m/m] (nicht nachweisbar)

**Beispiel 10**

**Tris-(3,6-dimethylpyrazin-2-ol-1-oxid)-eisen(III)-Komplex**

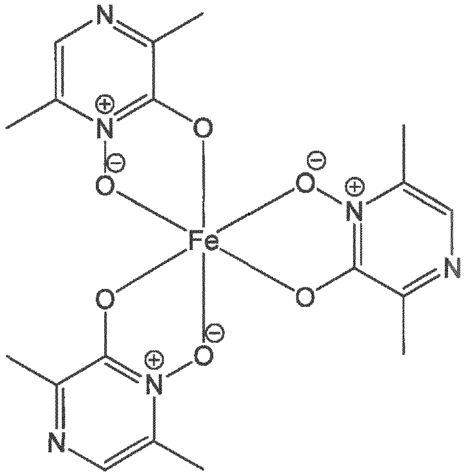

15 mmol (2.1 g) 3,6-Dimethylpyrazin-2-ol-1-oxid wurden in 50 ml Ethanol auf Rückfluss erhitzt und 5 mmol (1.35 g) in 10 ml Ethanol gelöstes $FeCl_3$*$6H_2O$ zugegeben. Unter Rückfluss wurden 50 ml Wasser zugetropft, 90 ml Lösungsmittel abdestilliert und die Reaktionsmischung abkühlen gelassen. Anschließend wurde mit 12 ml 1 M NaOH auf pH 4.4 eingestellt, das Produkt abfiltriert und getrocknet. Man erhielt 2.2 g (87% Fe-Ausbeute) der Titelverbindung.
IR (in Substanz, $cm^{-1}$): 1599, 1529, 1486, 1455, 1413, 1370, 1316, 1252, 1171, 1123, 1028, 978, 940, 859, 752, 707.
CHN-Elementaranalyse: C, 45.28; H, 4.31; N, 17.33.
Fe-Gehalt: 11.04% [m/m]
Chlorid-Gehalt: 0.0% [m/m] (nicht nachweisbar)

**PHARMAKOLOGISCHE TESTMETHODE:**

Die hervorragenden Fe-Utilisationen, die durch die erfindungsgemäßen Fe-Komplexe, erreicht werden können, wurden durch folgendes Mäuse-Modell gemessen.
Männliche NMRI (SPF)-Mäuse (ca. 3 Wochen alt) wurden ca. 3 Wochen mit eisenarmer Diät (ca. 5 ppm Eisen) gefüttert. Dann wurde jeweils 6 Mäusen während 2 mal 5 Tagen mit einer Unterbrechung von 2 Tagen (Tage 1 - 5 und 8 - 12) die Eisenkomplexe mittels Schlundsonde verabreicht (2 mg Eisen/kg Körpergewicht/Tag). 6 Mäuse dienten als Kontrollgruppe (Negativkontrolle) und erhielten stattdessen Wasser. Die Utilisation am Tag 15 wurde berechnet aus der Hämoglobinzunahme und der Körpergewichtszunahme nach der Formel

$$\text{Utilisation (\%)} = \frac{\Delta\,\text{Eisenutilisation} * 100}{\text{Fe Dos.}} = \frac{(\text{Fe ut.} - \text{Fe ut.Control}) * 100}{\text{Fe Dos.}}$$

$$= [(Hb_{2(3)} * BW_{9(14)} - Hb_1 * BW_4) * 0{,}07 * 0{,}0034 - (Hb_{2(3)\,Control} * BW_{9(14)\,Control} - Hb_{1\,Control} * BW_{4\,Control}) * 0{,}07 * 0{,}0034)] * 100/\ \text{Fe Dos.}$$

$$= [(Hb_{2(3)} * BW_{9(14)} - Hb_1 * BW_4) * 0{,}000238 - (Hb_{2(3)\,Control} * BW_{9(14)\,Control} - Hb_{1\,Control} * BW_{4\,Control}) * 0{,}000238] * 100 / \text{Fe Dos.}$$

$$= (Hb_{2(3)} * BW_{9(14)} - Hb_1 * BW_4 - Hb_{2(3)\,Control} * BW_{9(14)\,Control} + Hb_{1\,Control} * BW_{4\,Control}) * 0{,}0238 / \text{Fe Dos.}$$

| | |
|---|---|
| 0.07 | = Faktor für 70 ml Blut per kg Körpergewicht (BW) |
| 0.0034 | = Faktor für 0,0034 g Fe/g Hb |
| $Hb_1$ | = Hämoglobin-Wert (g/l) am Tag 1 |
| $Hb_{2(3)}$ | = Hämoglobin-Wert (g/l) am Tag 8 (oder 15) |
| $BW_4$ | = Körpergewicht (g) am Tag 1 |
| $BW_{9(14)}$ | = Körpergewicht (g) am Tag 8 (oder 15) |
| $Hb_{1\,Control}$ | = Durchschnitts-Hämoglobinwert (g/l) am Tag 1 in der Kontrollgruppe, |
| $Hb_{2(3)\,Control}$ | = Durchschnitts-Hämoglobinwert (g/l) am Tag 8 (oder 15) in der Kontrollgruppe, |
| $BW_{4\,Control}$ | = Durchschnittskörpergewicht (g) am Tag 1 in der Kontrollgruppe, |
| $BW_{9(14)\,Control}$ | = Durchschnittskörpergewicht (g) am Tag 8 (oder 15) in der Kontrollgruppe, |
| Fe Dos. | = Gesamtes verabreichtes Eisen (mg Fe) während 5 oder 10 Tage, |
| Fe ut. | = $(Hb_{2(3)} * BW_{9(14)} - Hb_1 * BW_4) * 0.07 * 0.0034$ (mg Fe) |
| Δ Utilisation = | Fe tot. utilisiert (Untersuchte Gruppe) - Fe ut. Kontrollgruppe, utilisiert aus Nahrung, (mg Fe). |

Die nachfolgende Tabelle zeigt die Ergebnisse:

Tabelle:

| Beispiel-Nr. | Utilisation n 15 d (abs. %) | Standardabweichung (+/- 0,5) |
|---|---|---|
| 1 | 56 | 13 |
| 2 | 61 | 11 |
| 3 | 77 | 15 |
| 4 | 32 | 15 |
| 5 | 37 | 7 |
| 10 | 58 | 16 |
| Vergleichsbeispiel* | 25 | 13 |

***Vergleichsbeispiel:**

Zum Vergleich wurde die Tris(pyridinon-2-ol 1-oxid)-Eisen(III)-Komplexverbindung der Formel:

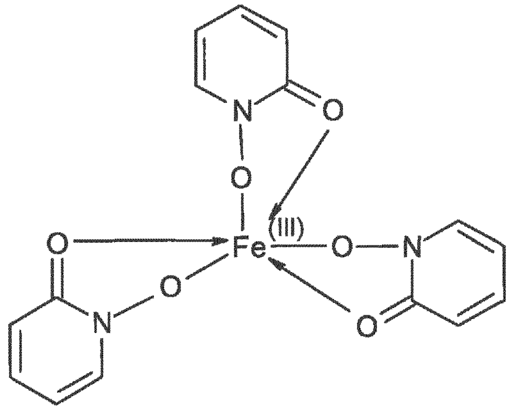

analog zur EP 0138420 hergestellt und getestet, um den Einfluß des heterocyclischen Grundkörpers zu belegen. EP 0138420 offenbart in Beispiel 7 lediglich Tris(pyridinon-2-ol 1-oxid)-Eisen(III)-Komplexverbindungen, die am Pyridinring einen weiteren Substituenten tragen. Die unsubstituierte, hier zum Vergleich herangezogene Tris(pyridinon-2-ol 1-oxid)-Eisen(III)-Komplexverbindung wird dort nicht offenbart. Wie die Ergebnisse in vorstehender Tabelle zeigen, weist die entsprechende erfindungsgemäße Pyrimidin-Verbindung des Beispiels 1 gegenüber der Pyridin-Vergleichsverbindung analog EP 0138420 eine deutlich verbesserte Eisen-Utilisation auf. Auch die erfindungsgemäßen Beispielverbindungen 2, 3, 4, 5 und 10 zeigen eine verbesserte Eisen-Utilisation gegenüber der Vergleichssubstanz.

Die gemessenen Eisen-Utilisationswerte stellen einen wichtigen Parameter im Hinblick auf die Indikation der Behandlung von Eisenmangelerscheinungen und Eisenmangelanämien dar, denn dieser Parameter spiegelt nicht nur die Eisenaufnahme, sondern auch den Zusammenhang zwischen Körpergewicht und Eisenaufnahme wieder, was gerade bei der Verwendung von heranwachsenden Tieren im Tiermodell wichtig ist. Würde man nur die Hämoglobin-Werte betrachten, die ein Maß für das wirklich aufgenommene und verwendete Eisen darstellen, würde man den auf dem Wachstum der Tiere beruhenden Anteil unberücksichtigt lassen. Somit stellt die Eisen-Utilisation eine genauere Meßgröße dar, obwohl Eisenutilisation und Hämoglobinwerte meist miteinander korrelieren. Eine Betrachtung des reinen Eisen-Serumspiegels, welche ebenfalls messbar wäre, kommt weniger in Betracht, da zwar eine Aussage über die Menge Eisen getroffen wird, die in den Körper gelangt, aber nicht darüber, wieviel der Körper davon auch nutzen kann.

Die Erfindung wird durch nachfolgenden Ausführungsformen weiter verdeutlicht:

1. Eisen(III)-pyrazin-2-ol 1-oxid-Komplexverbindungen oder deren pharmazeutisch verträgliche Salze zur Verwendung als Arzneimittel.

2. Eisen(III)-Komplexverbindungen nach Ausführungsform 1, enthaltend mindestens einen Liganden der Formel (I):

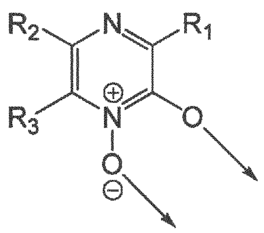

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- Halogen,
- gegebenenfalls substituiertem Alkyl,
- gegebenenfalls substituiertem Alkoxy,
- gegebenenfalls substituiertem Aryl,
- gegebenenfalls substituiertem Alkoxycarbonyl, und
- gegebenenfalls substituiertem Aminocarbonyl ist, oder

$R_2$ und $R_3$ gemeinsam mit den Kohlenstoffatomen, an die sie gebunden sind, einen gegebenenfalls substituierten 5- oder 6-gliedrigen gesättigten oder ungesättigten Ring bilden, der gegebenenfalls ein oder mehrere Heteroatome aufweisen kann,
oder pharmazeutisch verträgliche Salze davon.

3. Eisen(III)-Komplexverbindungen zur Verwendung nach einer oder mehrerer der Ausführungsformen 1 bis 2, enthaltend mindestens einen Liganden der Formel (I):

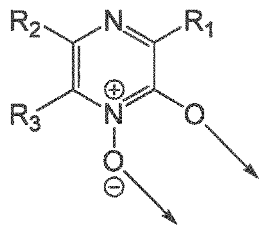

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- gegebenenfalls substituiertem Alkyl, und
- Halogen, oder

$R_2$ und $R_3$ gemeinsam mit den Kohlenstoffatomen, an die sie gebunden sind, einen 5- oder 6-gliedrigen carbocyclischen Ring bilden,
oder pharmazeutisch verträgliche Salze davon.

4. Eisen(III)-Komplexverbindungen zur Verwendung nach einer oder mehrerer der Ausführungsformen 1 bis 3, enthaltend mindestens einen Liganden der Formel (I):

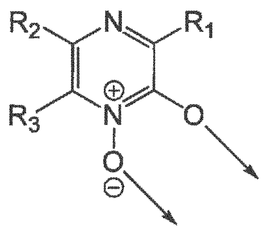

(I)

worin
die Pfeile jeweils eine koordinative Bindung zu einem oder verschiedenen Eisenatomen darstellen,
$R_1$, $R_2$, $R_3$ gleich oder verschieden sind und ausgewählt werden aus der Gruppe, die besteht aus Wasserstoff und Alkyl, das gegebenenfalls durch Alkoxy substituiert sein kann, oder
$R_2$ und $R_3$ bilden zusammen eine Propylen ($-CH_2-CH_2-CH_2-$), Butylen ($-CH_2-CH_2-CH_2-CH_2-$), Azabutylen oder Oxabutylen- Gruppe,
oder pharmazeutisch verträgliche Salze davon.

5. Eisen(III)-Komplexverbindungen zur Verwendung nach einer oder mehrerer der Ausführungsformen 1 bis 4, welche die folgende Formel aufweisen:

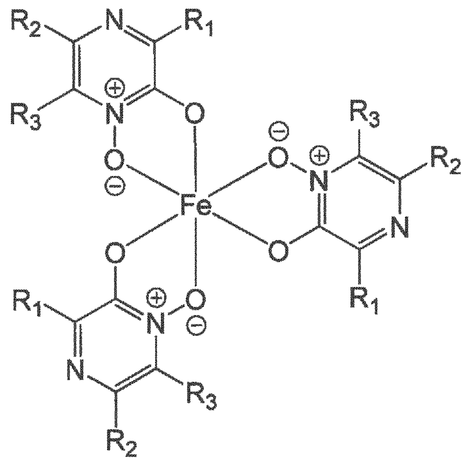

, (II)

worin $R_1$, $R_2$, $R_3$ jeweils gleich oder verschieden sein können und wie oben definiert sind, und pharmazeutisch verträgliche Salze davon.

6. Eisen(III)-pyrazin-2-ol 1-oxid-Komplexverbindungen, wie in einer der Ausführungsformen 1 bis 5 definiert, in fester Form.

7. Eisen(III)-pyrazin-2-ol 1-oxid-Komplexverbindungen nach Ausführungsform 6 zur Verwendung als Arzneimittel.

8. Eisen(III)-Komplexverbindungen zur Verwendung nach einer der Ausführungsformen 1 bis 5 sowie 7 zur Verwendung in der Behandlung und Prophylaxe vonEisenmangelerscheinungen und Eisenmangelanämien sowie der damit einhergehenden Symptome.

9. Eisen(III)-Komplexverbindungen zur Verwendung nach Ausführungsform 8, worin die Symptome einschließen: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen.

10. Eisen(III)-Komplexverbindungen zur Verwendung nach Ausführungsform 8 zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3-5 (CDK 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases).

11. Eisen(III)-Komplexverbindungen zur Verwendung nach einer der Ausführungsformen 1 bis 5 sowie 7 bis 10,worin die Eisen(III)-Komplexverbindung oral verabreicht wird.

12. Eisen(III)-Komplexverbindungen zur Verwendung nach Ausführungsform 11, worin die Verabreichung in Form

einer Tablette oder Kapsel, einschließlich säureresistenter Formen oder Formen mit pH-abhängigen Beschichtungen erfolgt.

13. Arzneimittel, enthaltend Eisen(III)-Komplexverbindungen wie in einer der Ausführungsformen 1 bis 6 definiert.

14. Arzneimittel, enthaltend Eisen(III)-Komplexverbindungen wie in einer der Ausführungsformen 1 bis 6 definiert sowie mindestens einen physiologisch verträglichen Träger bzw. Exzipienten.

15. Zusammensetzung enthaltend Eisen(III)-Komplexverbindungen, wie in einer der Ausführungsformen 1 bis 6 definiert, in Kombination mit mindestens einem weiteren Arzneimittel welches auf den Eisenmetabolismus wirkt.

## Patentansprüche

**1.** Eisen(III)-pyrazin-2-ol 1-oxid-Komplexverbindungen oder deren pharmazeutisch verträgliche Salze zur Verwendung in der Behandlung und Prophylaxe von Eisenmangelerscheinungen und Eisenmangelanämien sowie der damit einhergehenden Symptome.

**2.** Eisen(III)-Komplexverbindungen zur Verwendung nach Anspruch 1, enthaltend mindestens einen Liganden der Formel (I):

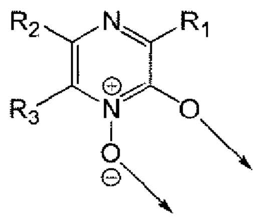

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- Halogen,
- gegebenenfalls substituiertem Alkyl,
- gegebenenfalls substituiertem Alkoxy,
- gegebenenfalls substituiertem Aryl,
- gegebenenfalls substituiertem Alkoxycarbonyl, und
- gegebenenfalls substituiertem Aminocarbonyl ist, oder

$R_2$ und $R_3$ gemeinsam mit den Kohlenstoffatomen, an die sie gebunden sind, einen gegebenenfalls substituierten 5- oder 6-gliedrige gesättigten oder ungesättigten Ring bilden, der gegebenenfalls ein oder mehrere Heteroatome aufweisen kann,
oder pharmazeutisch verträgliche Salze davon.

**3.** Eisen(III)-Komplexverbindungen zur Verwendung nach einem oder mehreren der Ansprüche 1 bis 2, enthaltend mindestens einen Liganden der Formel (I):

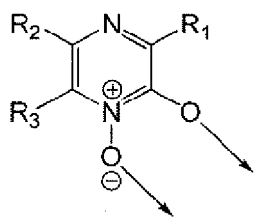

(I)

worin
die Pfeile jeweils eine koordinative Bindung zu einem oder verschiedenen Eisenatomen darstellen, und
$R_1$, $R_2$, $R_3$ gleich oder verschieden sein können und ausgewählt werden aus der Gruppe, die besteht aus:

- Wasserstoff,
- gegebenenfalls substituiertem Alkyl, und
- Halogen, oder

$R_2$ und $R_3$ gemeinsam mit den Kohlenstoffatomen, an die sie gebunden sind, einen 5- oder 6-gliedrigen carbocyclischen Ring bilden,
oder pharmazeutisch verträgliche Salze davon.

**4.** Eisen(III)-Komplexverbindungen zur Verwendung nach einem der Ansprüche 1 bis 3, enthaltend mindestens einen Liganden der Formel (I):

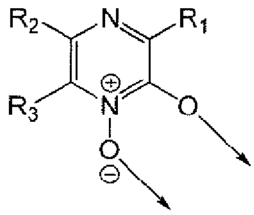

(I)

worin
die Pfeile jeweils eine koordinative Bindung zu einem oder verschiedenen Eisenatomen darstellen,
$R_1$, $R_2$, $R_3$ gleich oder verschieden sind und ausgewählt werden aus der Gruppe, die besteht aus Wasserstoff und Alkyl, das gegebenenfalls durch Alkoxy substituiert sein kann, oder
$R_2$ und $R_3$ bilden zusammen eine Propylen ($-CH_2-CH_2-CH_2-$), Butylen ($-CH_2-CH_2-CH_2-CH_2-$), Azabutylen oder Oxabutylen- Gruppe,
oder pharmazeutisch verträgliche Salze davon.

**5.** Eisen(III)-Komplexverbindungen zur Verwendung nach einem der Ansprüche 1 bis 4, welche die folgende Formel aufweisen:

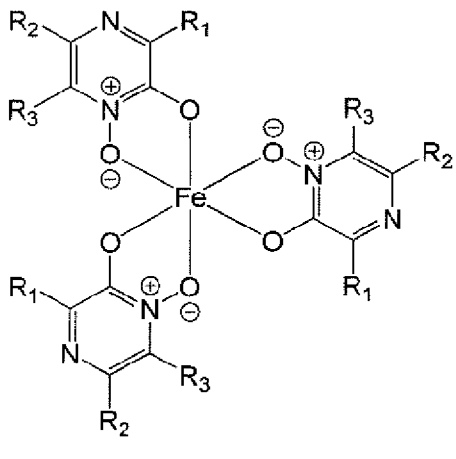

, (II)

worin $R_1$, $R_2$, $R_3$ jeweils gleich oder verschieden sein können und wie oben definiert sind, und pharmazeutisch verträgliche Salze davon.

**6.** Eisen(III)-Komplexverbindungen zur Verwendung nach einem der Ansprüche 1 bis 5, in fester Form.

7. Eisen(III)-Komplexverbindungen zur Verwendung nach einem der Ansprüche 1 bis 6, worin die Symptome einschließen: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen.

8. Eisen(III)-Komplexverbindungen zur Verwendung nach einem der Ansprüche 1 bis 6 zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangefanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3-5 (CKD 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases).

9. Eisen(III)-Komplexverbindungen zur Verwendung nach einem der Ansprüche 1 bis 8, worin die Eisen(III)-Komplexverbindung oral verabreicht wird.

10. Eisen(III)-Komplexverbindungen zur Verwendung nach Anspruch 9, worin die Verabreichung in Form einer Tablette oder Kapsel, einschließlich säureresistenter Formen oder Formen mit pH-abhängigen Beschichtungen erfolgt.

11. Eisen(III)-Komplexverbindungen zur Verwendung nach Anspruch 9 oder 10 zur Verabreichung in Form einer pharmazeutischen Zusammensetzung die mindestens einen physiologisch verträglichen Träger bzw. Exzipienten enthält.

12. Zusammensetzung enthaltend Eisen(III)-Komplexverbindungen, wie in einem der Ansprüche 1 bis 6 oder 11 definiert, in Kombination mit mindestens einem weiteren Arzneimittel welches auf den Eisenmetabolismus wirkt.

## Claims

1. Iron(III)-pyrazine-2-ol-1-oxide complex compounds or pharmaceutically acceptable salts thereof for use in the treatment and prophylaxis of iron deficiency symptoms and iron deficiency anemias and the symptoms associated therewith.

2. Iron(III)-complex compounds for use according to claim 1, containing at least one ligand of the formula (I):

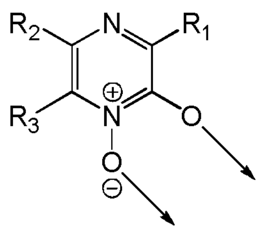

(I)

wherein
the arrows respectively represent a coordinate bond to one or different iron atoms, and
$R_1$, $R_2$, $R_3$ may be the same or different and are selected from the group consisting of:

- hydrogen,

- halogen,
- optionally substituted alkyl,
- optionally substituted alkoxy,
- optionally substituted aryl,
- optionally substituted alkoxycarbonyl, and
- optionally substituted aminocarbonyl or

$R_2$ and $R_3$ together with the carbon atoms to which they are bonded, form an optionally substituted saturated or unsaturated 5- or 6-membered ring, which may optionally contain one or more heteroatoms, or pharmaceutically acceptable salts thereof.

**3.** Iron(III)-complex compounds for use according to any one of claims 1 to 2, containing at least one ligand of the formula (I):

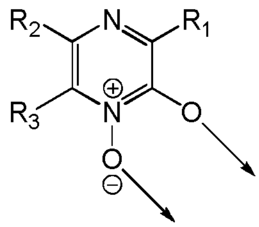

(I)

wherein
the arrows respectively represent a coordinate bond to one or different iron atoms, and
$R_1$, $R_2$, $R_3$ may be the same or different and are selected from the group consisting of:

- hydrogen,
- optionally substituted alkyl, and
- halogen, or

$R_2$ and $R_3$ together with the carbon atoms to which they are bonded, form a 5-or 6-membered carbocyclic ring, or pharmaceutically acceptable salts thereof.

**4.** Iron(III)-complex compounds for use according to any one of claims 1 to 3, containing at least one ligand of the formula (I):

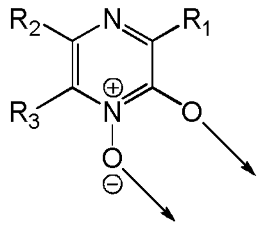

(I)

wherein
the arrows respectively represent a coordinate bond to one or different iron atoms, and
$R_1$, $R_2$, $R_3$ may be the same or different and are selected from the group consisting of hydrogen and alkyl, which may optionally be substituted by alkoxy, or
$R_2$ and $R_3$ together form a propylene group ($-CH_2-CH_2-CH_2-$), a butylene group ($-CH_2-CH_2-CH_2-CH_2-$), an azabutylene group or an oxabutylene group,
or pharmaceutically acceptable salts thereof.

**5.** Iron(III)-complex compounds for use according to any one of claims 1 to 4, represented by the following formula:

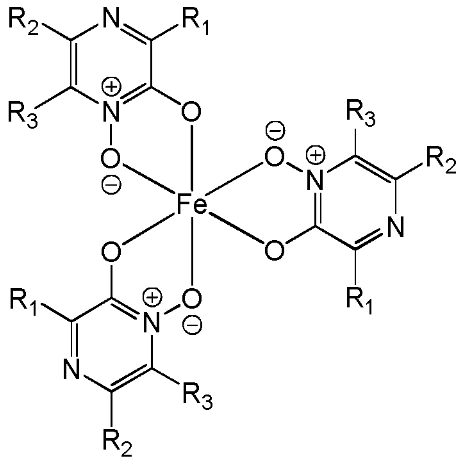

(II)

wherein $R_1$, $R_2$, $R_3$ may be the same or different and are defined as above, and pharmaceutically acceptable salts thereof.

**6.** Iron(III)-complex compounds for use according to any one of claims 1 to 5, being in solid form.

**7.** Iron(III) complex compounds for use according to any one of claims 1 to 6, wherein the symptoms include: fatigue, listlessness, lack of concentration, low cognitive efficiency, difficulties in finding the right words, forgetfulness, unnatural pallor, irritability, acceleration of heart rate (tachycardia), sore or swollen tongue, enlarged spleen, desire for strange foods (pica), headaches, lack of appetite, increased susceptibility to infections, depressive moods.

**8.** Iron(III) complex compounds for use according to any one of claims 1 to 6 for the treatment of iron deficiency anemia in pregnant women, latent iron deficiency anemia in children and adolescents, iron deficiency anemia caused by gastrointestinal abnormalities, iron deficiency anemia due to blood loss, such as gastrointestinal hemorrhage (e.g. due to ulcers, carcinoma, hemorrhoids, inflammatory disorders, taking of acetylsalicylic acid), iron deficiency anemia caused by menstruation, iron deficiency anemia caused by injuries, iron deficiency anemia due to psilosis (sprue), iron deficiency anemia due to reduced dietary iron uptake, in particular in selectively eating children and adolescents, immunodeficiency caused by iron deficiency anemia, brain function impairment caused by iron deficiency anemias, restless leg syndrome caused by iron deficiency anemias, iron deficiency anemias in the case of cancer, iron deficiency anemias caused by chemotherapies, iron deficiency anemias triggered by inflammation (AI), iron deficiency anemias in the case of congestive cardiac insufficiency (CHF; congestive heart failure), iron deficiency anemias in the case of chronic renal insufficiency stage 3-5 (CKD 3-5; chronic kidney diseases stage 3-5), iron deficiency anemias triggered by chronic inflammation (ACD), iron deficiency anemias in the case of rheumatoid arthritis (RA; rheumatoid arthritis), iron deficiency anemias in the case of systemic lupus erythematosus (SLE; systemic lupus erythematosus) and iron deficiency anemias in the case of inflammatory bowel diseases (IBD; inflammatory bowel diseases).

**9.** Iron(III) complex compounds for use according to any one of claims 1 to 8, wherein the iron(III) complex compound is administered orally.

**10.** Iron(III) complex compounds for use according to claim 9, which is administered in the form of a tablet or a capsule, including enteric coated forms or forms with pH-dependent coatings.

**11.** Iron(III) complex compounds for use according to claim 9 or 10 for administration in the form of a pharmaceutical preparation containing at least one physiological compatible carrier or excipient.

**12.** Composition containing iron(III) complex compounds as defined in any one of claims 1 to 6 or 11, in combination with at least one further medicament which acts on the iron metabolism.

**Revendications**

1. Composés de coordination de pyrazine-2-ol 1-oxyde de fer(III) ou les sels pharmaceutiquement acceptables de ceux-ci à l'utilisation pour le traitement et la prophylaxie des apparitions de la carence en fer et des anémies par carence en fer ainsi que des symptômes qui y sont liés.

2. Composés de coordination de fer(III) à l'utilisation selon la revendication 1, contenant au moins un ligand de la formule (I):

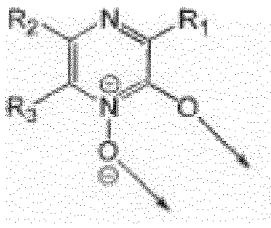
(I)

dans laquelle
les flèches chacune représentent une liaison coordinatrice avec un ou de différents atomes de fer, et
$R_1$, $R_2$, $R_3$ peuvent être égaux ou différents et sont sélectionnés parmi le groupe, qui consiste en :

hydrogène,
halogène,
alkyle, éventuellement substitué,
alkoxy, éventuellement substitué,
aryle, éventuellement substitué,
alkoxycarbonyle, éventuellement substitué, et
aminocarbonyle, éventuellement substitué, ou

$R_2$ et $R_3$ ensemble avec les atomes des carbone, auxquels ils sont liés, forment un anneau saturé ou non-saturé à 5 ou 6 chaînons éventuellement substitué, qui éventuellement peut avoir un ou plusieurs hétéroatomes,
ou les sels pharmaceutiquement acceptables de ceux-ci.

3. Composés de coordination de fer(III) à l'utilisation selon l'une ou plusieurs des revendications 1 à 2, contenant au moins un ligand de la formule (I):

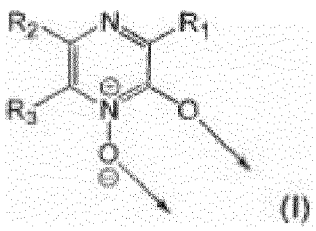
(I)

dans laquelle
les flèches chacune représentent une liaison coordinatrice avec un ou de différents atomes de fer, et $R_1$, $R_2$, $R_3$ peuvent être égaux ou différents et sont sélectionnés parmi le groupe, qui consiste en :

hydrogène,
alkyle éventuellement substitué, et
halogène, ou

$R_2$ et $R_3$ ensemble avec les atomes des carbone, auxquels ils sont liés, forment un anneau carboxylique à 5 ou 6 chaînons,
ou les sels pharmaceutiquement acceptables de ceux-ci.

4. Composés de coordination de fer(III) à l'utilisation selon l'une des revendications 1 à 3, contenant au moins un ligand de la formule (I):

(I)

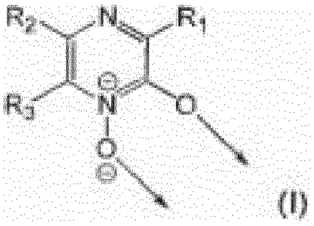

dans laquelle
les flèches chacune représentent une liaison coordinatrice avec un ou de différents atomes de fer,
$R_1$, $R_2$, $R_3$ sont égaux ou différents et sont sélectionnés parmi le groupe, qui consiste en hydrogène et alkyle, qui éventuellement peut être substitué par alkoxy, ou $R_2$ et $R_3$ ensemble forment un groupe propylène (-CH2-CH2-CH2-), butylène, (-CH2CH2CH2CH2-), azabutylène ou oxabutylène, ou les sels pharmaceutiquement acceptables de ceux-ci.

5. Composés de coordination de fer(III) à l'utilisation selon l'une des revendications 1 à 4, qui a la formule suivante:

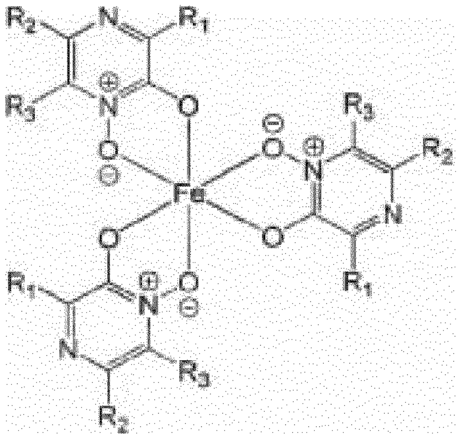

(II)

dans laquelle
chacun de $R_1$, $R_2$, $R_3$ peuvent être égaux ou différents et sont tels que définis ci-dessus, et les sels pharmaceutiquement acceptables de ceux-ci.

6. Composés de coordination de fer(III) à l'utilisation selon l'une des revendications 1 à 5, en forme solide.

7. Composés de coordination de fer(III) à l'utilisation selon l'une des revendications 1 à 6, dans laquelle les symptômes comprennent : la fatigue, l'absence d'énergie, les difficultés de concentration, l'efficacité cognitive faible, les difficultés à trouver le mots justes, l'oubli, la pâleur antinaturelle, l'irritabilité, l'augmentation du rythme cardiaque (la tachycardie), la langue endolorie ou gonflée, la rate agrandie, les fringales de grossesse (la maladie de Pica), les maux de tête, la manque d'appétit, la prédisposition accrue aux infections, les états dépressifs.

8. Composés de coordination de fer(III) à l'utilisation selon l'une des revendications 1 à 6 pour le traitement de l'anémie par carence en fer des femmes enceintes, de l'anémie par carence en fer latente chez les enfants et les adolescents, de l'anémie par carence en fer latente suite à des anormalités gastro-intestinaux, de l'anémie par carence en fer suite à des pertes de sang, tel que par les saignement gastro-intestinaux (ex. en raison des ulcères, des carcinomes, des hémorroïdes, des troubles inflammatoires, de la prise de acide acétylsalicylique), de l'anémie par carence en fer suite à la menstruation, de l'anémie par carence en fer suite aux lésions, de l'anémie par carence en fer à la

suite de psilose (sprue), de l'anémie par carence en fer suite à l'ingestion réduite de fer avec l'alimentation, surtout chez les enfants et les adolescents mangeant sélectivement, de l'immunodéficience provoquée par l'anémie par carence en fer, de la déficience de la performance cérébrale provoquée par les anémies par carence en fer, du syndrome des jambes sans repos provoqué par les anémies par carence en fer, les anémies par carence en fer chez le cancer, les anémies par carence en fer provoquées par les chimiothérapies, les anémies par carence en fer provoquées par l'inflammation (AI), les anémies par carence en fer au cours de l'insuffisance cardiaque congestive (CHF; congestive heart failure), les anémies par carence en fer au cours de l'insuffisance rénale chronique stade 3-5 (CKD 3-5; chronic kidney diseases stage 3-5), les anémies par carence en fer provoquées par l'inflammation chronique (ACD), les anémies par carence en fer au cours de l'arthrite rhumatoïde (RA; rheumatoid arthritis), les anémies par carence en fer au cours du lupus érythémateux systémique (SLE; systemic lupus erythematosus) et les anémies par carence en fer au cours des maladies inflammatoires de l'intestin (IBD, inflammatory bowel diseases).

**9.** Composés de coordination de fer(III) à l'utilisation selon l'une des revendications 1 à 8, dans laquelle le composé de coordination de fer(III) est administré par voie orale.

**10.** Composés de coordination de fer(III) à l'utilisation selon la revendication 9, dans laquelle l'administration est mise en oeuvre en forme d'une comprimé ou gélule, y compris les formes résistantes à l'acide ou les formes avec des couches dépendantes du pH.

**11.** Composés de coordination de fer(III) à l'utilisation selon la revendication 9 ou 10 pour l'administration en forme d'une composition pharmaceutique qui contient au moins un support ou excipient physiologiquement acceptable, respectivement.

**12.** Composition contenant des composés de coordination de fer(III), tels que définis selon l'une des revendications 1 à 6 ou 11, en association avec au moins un autre médicament, qui agit sur le métabolisme du fer.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 20081455586 A
- WO 2007062546 A
- WO 20040437865 A
- US 2003236224 A
- EP 150085 A
- CN 101481404
- EP 939083 A
- JP 02083400 A
- US 474670 A
- CN 1687089
- US 2009035385 A
- EP 308362 A
- ES 2044777
- EP 159194 A
- EP 138420 A
- EP 107458 A
- EP 0120670 A
- WO 2006037449 A
- GB 842637 A
- EP 0138420 A


### In der Beschreibung aufgeführte Nicht-Patentliteratur


- Balancing acts: molecular control of mammalian iron metabolism. **M.W. HENTZE.** Cell. 2004, vol. 117, 285-297
- *Biometals,* 2009, vol. 22, 701-710
- *INTERNATIONAL JOURNAL FOR VITAMIN AND NUTRITION RESEARCH,* 2007, vol. 77 (1), ISSN 0300-9831, 13-21
- *Bull. Chem. Soc. Jpn.,* 1993, vol. 66, 841-841
- *Reviews On Heteroatom Chemistry,* 1998, vol. 18, 87-118
- TETRAHEDRON. ELSEVIER SCIENCE PUBLISHERS, 20. November 1995, vol. 51, 12995-13002
- *J. Org. Chem.,* 1997, vol. 62, 3618-3624
- *J. Am. Chem. Soc.,* 1985, vol. 107, 6540-6546
- *Inorganica Chimica Acta,* 1987, vol. 135, 145-150
- *NATURAL PRODUCT COMMUNICATIONS,* 2011, vol. 6 (8), 1137-1140
- *Journal of Agricultural and Food Chemistry,* 01. Januar 1981, 785-787
- *Chemical & Pharmaceutical Bulletin,* 01. Januar 1978, 1320-1322
- *Chemical & Pharmaceutical Bulletin,* 01. Januar 1994, 277-279
- *Chemical & Pharmaceutical Bulletin,* 01. Januar 1981, 1510-1517
- HETEROCYCLES. ELSEVIER SCIENCE PUBLISHERS, 01. Januar 1993, vol. 35, 1279-1287
- *Natural Product Communications,* 2011, vol. 6 (8), 1137-1140
- *J. Org. Chem,* 1997, vol. 62, 3618-3624
- *J. Chem. Soc.,* 1949, 2707-2712
- **OHKANDA.** *Bull. Chem. Soc. Jpn,* 1993, vol. 66, 841-847
- *J. Org. Chem.,* 1958, vol. 23, 1603-1606
- *J. Heterocycl. Chem.,* 1983, vol. 19, 1061
- *J. Heterocycl. Chem.,* 1989, vol. 26, 812
- **G. DUNN et al.** *J. Chem. Soc.,* 1949, 2707-2712
- **C.E. MIXAN ; R. GARTH.** *J. Org. Chem.,* 1977, vol. 42, 1869-1871
- **A. OHTA et al.** *J. Heterocyclic Chem.,* 1981, vol. 18, 555-558